# EUROPEAN PATENT SPECIFICATION

(11) **EP 4 440 509 B1**
(45) Date of publication and mention of the grant of the patent: **02.12.2026**
(21) Application number: 22840467.9
(22) Date of filing: 29.11.2022
(51) Int. Cl.: A61F 2/958, A61F 2/24, A61M 25/10

(54) **CATHETER BALLOON HAVING SEGMENTS OF VARYING COMPLIANCE**
KATHETERBALLON MIT SEGMENTEN UNTERSCHIEDLICHER NACHGIEBIGKEIT
BALLONNET DE CATHÉTER AYANT DES SEGMENTS DE CONFORMITÉ VARIABLE

(30) Priority: 30.11.2021 US 202163264702 P
(43) Date of publication of application: 09.10.2024
(73) Proprietor: Edwards Lifesciences Corporation, Irvine, CA 92614 (US)
(72) Inventor: ZHU, Yidong, M., Irvine, CA 92614 (US); BULMAN, Erik, Irvine, CA 92614 (US)
(74) Representative: Eisenführ Speiser
(86) International application number: PCT/US2022/051159
(87) International publication number: WO 2023/101929

(56) References cited:
- WO-A2-2014/123983
- US-A1- 2012 065 718
- US-A1- 2013 253 466

## Description

### CROSS-REFERENCE TO RELATED APPLICATION

This application claims the benefit of U.S. Provisional Patent Application No. 63/264,702, filed November 30, 2021.

### FIELD

The present disclosure relates to inflatable balloons for balloon catheters, such as a delivery apparatus for a radially expandable medical device.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted.

WO 2014/123983 A2 discloses an inflatable medical device comprises a balloon having a constant diameter section, proximal and distal tapered sections, and proximal and distal stem sections, and a first layer over the balloon comprising a fiber extending circumferentially around the balloon, the fiber having a pitch in the proximal and distal tapered sections that is greater than a pitch of the fiber in the constant diameter and proximal and distal stem sections.

US 2013/0253466 A1 discloses a catheter balloon assembly comprising an inflatable balloon having a balloon body portion defining a balloon working length and an uninflated diameter and a working diameter, and a frangible cover covering at least a portion of the balloon body portion, the frangible cover being operable to rupture under an internal pressure before the rupture of the balloon wherein the frangible balloon cover is operable to control the balloon to open to an intermediate diameter that is less than the working diameter.

US 2012/0065718 A1 discloses a balloon catheter, comprising a) an elongated catheter shaft having a proximal end, a distal end, and an inflation lumen; and b) a multilayered balloon on a distal section of the shaft having an interior in fluid communication with the inflation lumen, and having a first layer of a first elastomeric polymeric material, and a second layer of a second elastomeric polymeric material having a lower blow up ratio (BUR) than the first material and being an outer layer relative to the first layer, the balloon being formed by a multistage blow-molding such that the balloon is formed in a first stage while limiting the balloon's radial growth, and then in a subsequent stage is blown to a final working radial dimension, where the multi-layer balloon has an area draw-down ratio (ADDR) that is reduced as compared to a multilayered balloon formed from a single stage.

### SUMMARY

Described herein are inflatable balloons for medical (balloon) catheters. In some examples, the inflatable balloons described herein can be used in a delivery apparatus for a prosthetic heart valve. Described herein are examples of such delivery apparatuses, prosthetic heart valves, and methods for implanting prosthetic heart valves. The disclosed balloons and methods for manufacturing the balloons can, for example, provide balloons with varying compliance segments that are configured such that the balloon ruptures in an axial direction under pressure from an inflation pressure received by the balloon. As such, the devices and methods disclosed herein can, among other things, overcome one or more of the deficiencies of typical delivery apparatuses.

A balloon catheter can comprise a handle, one or more shafts coupled to the handle, and an inflatable balloon mounted to the shaft.

In some examples, the balloon of the balloon catheter can comprise two or more varying compliance segments that are configured such that the balloon ruptures in an axial direction under pressure from an inflation pressure received by the balloon.

In some examples, the balloon of the balloon catheter can comprise two or more portions having different durometers, where one of the portions includes a discontinuity that extends in an axial direction along the balloon.

In some examples, an inflatable balloon for a medical catheter includes a first segment that has a first compliance and a second segment that has a second compliance. The first compliance is higher than the second compliance and the first segment and the second segment extend axially along a length of the balloon. The first segment is configured to rupture prior to the second segment in an axial direction under pressure from an inflation fluid introduced into the balloon.

In some examples, an inflatable balloon for a medical catheter includes a first circumferential segment comprising one or more layers of a first material with a first durometer, the one or more layers extending across a thickness of the balloon. The balloon further includes a second circumferential segment comprising one or more layers of the first material and one or more layers of a second material with a second durometer, the second durometer greater than the first durometer. The first circumferential segment is configured to form a rupture in an axial direction prior to the second circumferential segment under pressure from an inflation fluid introduced into the balloon.

In some examples, an inflatable balloon for a medical catheter includes a first portion having a first durometer, a second portion having a second durometer that is greater than the first durometer, and a discontinuity in the second portion, the discontinuity extending in an axial direction along a length of the balloon.

In some examples, a balloon catheter includes a shaft extending from a handle of the balloon catheter and an inflatable balloon mounted to the shaft. The balloon includes a plurality of axially extending circumferential segments that have different compliances, the plurality of segments configured such that a first segment having a higher compliance than a second segment is configured to rupture in an axial direction along the balloon before the second segment as the balloon is inflated with an inflation fluid.

In some examples, an inflatable balloon for a medical catheter includes an inner surface configured to contact a fluid used to inflate the balloon and one or more reinforcement elements disposed on the inner surface. Each reinforcement element extends in an axial direction along the balloon.

In some examples, a balloon catheter includes a shaft extending from a handle of the balloon catheter and an inflatable balloon mounted to the shaft. The balloon includes one or more reinforcement elements disposed on an inner surface of the balloon, each reinforcement element extending in an axial direction along the balloon and adding thickness to the balloon in a radial direction, at a selected circumferential position along an axial length of the balloon.

In some examples, a balloon and/or balloon catheter comprises one or more of the components recited in Examples 1-67 below.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 is a perspective view of a prosthetic heart valve, according to one example.
FIG. 2 is a perspective view of a delivery apparatus for a prosthetic heart valve, according to an example.
FIG. 3 is a perspective view of a balloon that has experienced an exemplary tear in a lateral direction.
FIG. 4 is an exemplary graph depicting an outer diameter of a balloon as inflation pressure of an inflation fluid received within the balloon increases, the graph depicting a threshold pressure for rupture for two materials of varying durometer in the balloon.
FIG. 5 is a cross-sectional view of an exemplary balloon comprising an axially extending circumferential segment that is configured to rupture in an axial direction before a remainder of the balloon under pressure from an inflation fluid introduced into the balloon.
FIG. 6 is a perspective view of the balloon of FIG. 5.
FIG. 7 is a cross-sectional view of an exemplary balloon comprising multiple axially-extending circumferential segments that are configured to rupture in an axial direction before a remainder of the balloon.
FIG. 8 is a cross-sectional view of an exemplary balloon comprising multiple axially-extending circumferential segments that are configured to rupture in an axial direction before a remainder of the balloon.
FIGS. 9A-9C are cross-sectional views of an exemplary balloon comprising an axially-extending circumferential segment that is configured to rupture in an axial direction before a remainder of the balloon, in various stages of inflation of the balloon depicting the larger growth of the circumferential segment compared to the remainder of the balloon which comprises a larger durometer material.
FIG. 10 is a cross-sectional view of an exemplary balloon comprising multiple axially-extending circumferential segments, formed by discontinuities in a higher durometer portion of the balloon, that are configured to rupture in an axial direction before a remainder of the balloon.
FIG. 11 is a cross-sectional view of an exemplary balloon comprising multiple axially-extending circumferential segments, formed by discontinuities in a higher durometer portion of the balloon, that are configured to rupture in an axial direction before a remainder of the balloon.
FIG. 12 is a cross-sectional view of an exemplary balloon comprising multiple axially-extending circumferential segments, formed by discontinuities in a higher durometer portion of the balloon, that are configured to rupture in an axial direction before a remainder of the balloon.
FIG. 13 is a cross-sectional view of an exemplary balloon comprising a discontinuity created by overlapping ends of a higher durometer portion of the balloon, the discontinuity forming an axially extending circumferential segment that is configured to rupture before a remainder of the balloon.
FIG. 14 is a cross-sectional view of an exemplary balloon comprising a discontinuity created by overlapping ends of a higher durometer portion of the balloon, the discontinuity forming an axially extending circumferential segment that is configured to rupture before a remainder of the balloon.
FIG. 15 is a cross-sectional view of an exemplary balloon comprising two portions having different durometers where a compliance or durometer variation in the circumferential direction of the balloon is created by altering a number of layers of a higher durometer portion of the two portions within the lower durometer portion of the two portions of the balloon.
FIG. 16 is a cross-sectional view of an exemplary balloon comprising a circumferentially extending higher durometer portion that varies in thickness in a circumferential direction and a circumferentially extending lower durometer portion surrounding the higher durometer portion such that axially extending circumferential segments that are configured to rupture before a remainder of the balloon are formed in regions of thinner sections of the higher durometer portion.
FIG. 17 is a cross-sectional view of an exemplary balloon comprising multiple axially-extending circumferential segments, formed by discontinuities in a higher durometer portion of the balloon, that are configured to rupture in an axial direction before a remainder of the balloon.
FIG. 18 is a cross-sectional view of an exemplary balloon comprising an axially-extending circumferential segment, formed by a discontinuity in a higher durometer portion of the balloon, that is configured to rupture in an axial direction before a remainder of the balloon.
FIG. 19 is a cross-sectional view of an exemplary balloon comprising an axially-extending circumferential segment, formed by discontinuities in one or more higher durometer portions of the balloon, that is configured to rupture in an axial direction before a remainder of the balloon.
FIG. 20 is a cross-sectional view of an exemplary balloon comprising an axially-extending circumferential segment formed by a discontinuity in a higher durometer portion of the balloon that is filled with a lower durometer material, the circumferential segment configured to rupture in an axial direction before a remainder of the balloon.
FIG. 21 is a cross-sectional view of an exemplary balloon comprising axially-extending circumferential segments formed by discontinuities in a higher durometer portion of the balloon that are filled with a lower durometer material, the circumferential segments configured to rupture in an axial direction before a remainder of the balloon.
FIG. 22 is a cross-sectional view of an exemplary balloon comprising an axially-extending circumferential segment, formed by a discontinuity in a higher durometer portion of the balloon, that is configured to rupture in an axial direction before a remainder of the balloon.
FIG. 23 is a cross-sectional view of an exemplary balloon comprising an axially-extending circumferential segment, formed by a discontinuity in one or more varying durometer layers of the balloon that is filled by a lower durometer material, the circumferential segment configured to rupture in an axial direction before a remainder of the balloon.
FIG. 24 is a cross-sectional view of an exemplary balloon comprising multiple layers of varying durometer portions of the balloon and an axially-extending circumferential segment that is formed by a discontinuity in a higher durometer portion, the circumferential segment configured to rupture in an axial direction before a remainder of the balloon.
FIG. 25 is a flow chart of a method for forming a balloon with one or more circumferential segments that have a higher compliance (and lower durometer) than a remaining portion of the balloon such that the one or more circumferential segments are configured to rupture in an axial direction before a remainder of the balloon under pressure from an inflation fluid introduced into the balloon.
FIG. 26 is a cross-sectional view of an exemplary extruded balloon tube, prior to forming into a balloon, the balloon tube comprising one of more axially extending reinforcement elements disposed on an inner circumferential surface of the balloon tube, the one or more reinforcement elements configured such that a balloon formed from the balloon tube is configured to rupture in an axial direction under a threshold pressure from an inflation fluid introduced into the balloon.
FIG. 27 is a cross-sectional view of a portion of a balloon formed from the balloon tube of FIG. 26.

### DETAILED DESCRIPTION

### General Considerations

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Overview of the Disclosed Technology

As introduced above, a prosthetic heart valve can be mounted in a crimped state on the distal end of a delivery apparatus and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted. **In** some instances, the balloon of the delivery apparatus can tear during an implantation procedure, such as from inadvertent overinflation. A tear in a lateral direction across the balloon can result in the balloon becoming caught (on the prosthetic heart valve or another type of expandable device being implanted with the delivery apparatus, or on the delivery apparatus) when removing the delivery apparatus from a body of a patient. This can increase a complexity and/or duration of the implantation procedure.

Accordingly, a need exists for improved balloons for delivery apparatuses or balloon catheters and methods of fabricating balloons for a delivery apparatus such that degradation or tears in the balloon that can cause the balloon to become stuck (e.g., lateral tears) are avoided.

Described herein are examples of inflatable balloons for balloon catheters, the balloons having two or more circumferential segments that vary in compliance. The variance in compliance between the two or more circumferential segments can result from varying durometer materials in the different circumferential segments. Each circumferential segment can extend axially along the balloon. A circumferential segment of the balloon that has a higher compliance (and lower durometer) than other circumferential segments of the balloon can be configured to rupture in the axial direction under pressure from an inflation fluid introduced into the balloon prior to a remainder of the balloon. In this way, a balloon can be configured such that ruptures in the axial direction occur when reaching a threshold inflation pressure, instead of in a lateral direction.

In some examples, as introduced above, the varying compliance circumferential segments can be created by utilizing different durometer materials to form the different circumferential segments.

In some examples, the varying compliance circumferential segments can be created with axially extending reinforcement elements disposed on an inner circumferential surface of the balloon, and outer circumferential surface of the balloon, or both the inner and outer circumferential surfaces of the balloon.

Also described herein are methods for forming inflatable balloons with two or more varying compliance and/or durometer segments.

Prosthetic valves disclosed herein can be radially compressible and expandable between a radially compressed state and a radially expanded state. Thus, the prosthetic valves can be crimped on or retained by an implant delivery apparatus in the radially compressed state during delivery, and then expanded to the radially expanded state once the prosthetic valve reaches the implantation site. It is understood that the prosthetic valves disclosed herein may be used with a variety of implant delivery apparatuses and can be implanted via various delivery procedures, examples of which will be discussed in more detail later.

### Examples of the Disclosed Technology

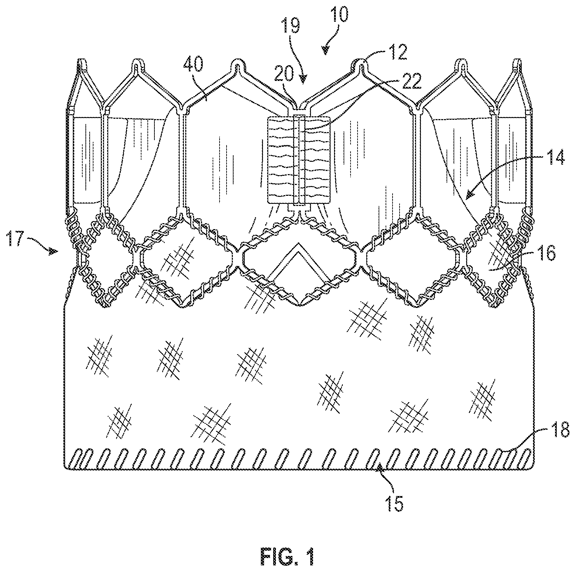

FIG. 1 shows an exemplary prosthetic valve 10, according to one example. Any of the prosthetic valves disclosed herein are adapted to be implanted in the native aortic annulus, although in some examples they can be adapted to be implanted in the other native annuluses of the heart (the pulmonary, mitral, and tricuspid valves). The disclosed prosthetic valves also can be implanted within vessels communicating with the heart, including a pulmonary artery (for replacing the function of a diseased pulmonary valve, or the superior vena cava or the inferior vena cava (for replacing the function of a diseased tricuspid valve) or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756. In some examples, the disclosed prosthetic valves can be implanted within a docking device implanted within or at the native mitral valve, such as disclosed in PCT Publication No. WO2020/247907. In some examples, the disclosed prosthetic valves can be implanted within a docking device implanted within the superior or inferior vena cava for replacing the function of a diseased tricuspid valve, such as disclosed in U.S. Publication No. 2019/0000615.

The prosthetic valve 10 can have four main components: a stent or frame 12, a valvular structure 14, an inner skirt 16, and a perivalvular outer sealing member or outer skirt 18. The prosthetic valve 10 can have an inflow end portion 15, an intermediate portion 17, and an outflow end portion 19.

The valvular structure 14 can comprise three leaflets 40, collectively forming a leaflet structure, which can be arranged to collapse in a tricuspid arrangement, although in other examples there can be greater or fewer number of leaflets (e.g., one or more leaflets 40). The leaflets 40 can be secured to one another at their adjacent sides to form commissures 22 of the valvular (e.g., leaflet) structure 14. The lower edge of valvular structure 14 can have an undulating, curved scalloped shape and can be secured to the inner skirt 16 by sutures (not shown). In some examples, the leaflets 40 can be formed of pericardial tissue (e.g., bovine pericardial tissue), biocompatible synthetic materials, or various other suitable natural or synthetic materials as known in the art and described in U.S. Patent No. 6,730,118.

The frame 12 can be formed with a plurality of circumferentially spaced slots, or commissure windows 20 that are adapted to mount the commissures 22 of the valvular structure 14 to the frame. The frame 12 can be made of any of various suitable plastically-expandable materials (e.g., stainless steel, etc.) or self-expanding materials (e.g., nickel titanium alloy (NiTi), such as nitinol), as known in the art. When constructed of a plastically-expandable material, the frame 12 (and thus the prosthetic valve 10) can be crimped to a radially collapsed configuration on a delivery catheter and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frame 12 include, without limitation, stainless steel, a biocompatible, high-strength alloys (e.g., a cobalt-chromium or a nickel-cobalt-chromium alloys), polymers, or combinations thereof. In particular examples, frame 12 is made of a nickel-cobalt-chromium-molybdenum alloy, such as MP35N^{®} alloy (SPS Technologies, Jenkintown, Pennsylvania), which is equivalent to UNS R30035 alloy (covered by ASTM F562-02). MP35N^{®} alloy/UNS R30035 alloy comprises 35% nickel, 35% cobalt, 20% chromium, and 10% molybdenum, by weight. Additional details regarding the prosthetic valve 10 and its various components are described in WIPO Patent Application Publication No. WO 2018/222799.

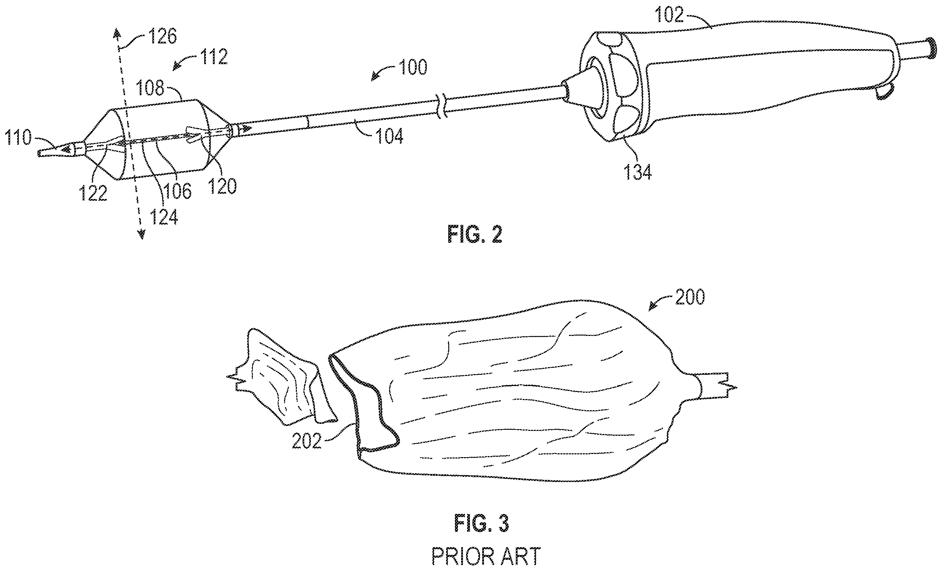

FIG. 2 shows a delivery apparatus 100, according to an example, that can be used to implant an expandable prosthetic heart valve (e.g., prosthetic valve 10 or 50), or another type of expandable prosthetic medical device (such as a stent). In some examples, the delivery apparatus 100 is specifically adapted for use in introducing a prosthetic valve into a heart.

The delivery apparatus 100 in the illustrated example of FIG. 2 is a balloon catheter comprising a handle 102, a steerable, outer shaft 104 extending from the handle 102, an intermediate shaft extending from the handle 102 coaxially through the steerable outer shaft 104, and an inner shaft 106 extending from the handle 102 coaxially through the intermediate shaft and the steerable, outer shaft 104, an inflatable balloon (e.g., balloon) 108 extending from a distal end of the intermediate shaft, and a nosecone 110 arranged at a distal end of the delivery apparatus 100. A distal end portion 112 of the delivery apparatus 100 includes the balloon 108, the nosecone 110, and a balloon shoulder assembly. A prosthetic medical device, such as a prosthetic heart valve may be mounted on a valve retaining portion of the balloon 108. A balloon shoulder assembly is configured to maintain the prosthetic heart valve or other medical device at a fixed position on the balloon 108 during delivery through the patient's vasculature. In some examples, the balloon shoulder assembly can include a proximal shoulder 120 and/or a distal shoulder 122.

The balloon 108 can include a central portion (which can be approximately cylindrical when inflated, as shown in FIG. 2) and two tapered end portions that connect to the delivery apparatus 100 (e.g., to one or more shafts and/or a nosecone of the delivery apparatus). A length of the balloon 108 can be defined in an axial direction 124 (which can be parallel to a central longitudinal axis of the delivery apparatus 100 and the balloon 108). Further, a lateral (or radial) direction 126 can be defined perpendicular to the axial direction 124.

The handle 102 can include a steering mechanism configured to adjust the curvature of the distal end portion of the delivery apparatus. In the illustrated example, for example, the handle 102 includes an adjustment member, such as the illustrated rotatable knob 134, which in turn is operatively coupled to the proximal end portion of a pull wire (not shown). The pull wire extends distally from the handle 102 through the outer shaft 104 and has a distal end portion affixed to the outer shaft at or near the distal end of the outer shaft 104. Rotating the knob 134 is effective to increase or decrease the tension in the pull wire, thereby adjusting the curvature of the distal end portion of the delivery apparatus.

The delivery apparatus 100 can be configured to be advanced over a guidewire that can be received within a guidewire lumen defined by an innermost shaft of the delivery apparatus 100.

In some examples, the delivery apparatus (or a similar delivery apparatus) can be configured to deploy and implant a prosthetic heart valve (e.g., prosthetic valve 10 of FIG. 1) in the native aortic annulus of a native aortic valve. Further details on such a delivery apparatus can be found in International Application No. PCT/US2021/047056.

As an example, during an implantation procedure for implanting an expandable prosthetic heart valve (e.g., prosthetic valve 10 of FIG. 1), the distal end portion of the delivery apparatus 100 (or a similar delivery apparatus or balloon catheter) can be advanced (over a guidewire) to a target implantation site. The balloon 108 can then be inflated to radially expand and implant the prosthetic heart valve at the implantation site.

In some examples, the balloon can rupture (e.g., tear) during the implantation procedure. As shown in the example of FIG. 3, a balloon 200 can experience a lateral tear 202. As used herein, a "lateral tear" can refer to a tear that occurs across the balloon 200 in a plane that is substantially perpendicular to a central longitudinal axis of the balloon 200 and that is defined by the radial and the circumferential directions (e.g., a tear in the lateral direction 126 shown in FIG. 2 or substantially in the lateral direction, such as laterally across an entire or partial portion of the balloon). Lateral tears, such as the lateral tear 202 shown in FIG. 3, can form a circumferentially extending torn edge on the balloon. As such, lateral tears, such as the exemplary lateral tear 202, can result in a portion of the torn balloon getting caught upon removal of the delivery apparatus 100 from the patient's vasculature. For example, the portion of the torn balloon may get caught on the prosthetic medical device (e.g., valve) or the delivery apparatus. Efforts to remove the caught portion of the balloon can increase procedure times and costs.

In some examples, it can be advantageous for the balloon to tear in the axial direction 124 (FIG. 2) or longitudinally along the balloon in a direction that is parallel to a central longitudinal axis of the balloon. An axial tear can form an axially extending torn edge along at least a portion of the balloon. Longitudinal or axial tears can make the torn balloon easier to retrieve since all portions of the torn balloon may remain connected to the balloon catheter (e.g., delivery apparatus 100 of FIG. 2).

By designing a balloon to have a variance in radial strength or a variance in strain on the balloon in a radial direction, the balloon can be designed to tear in the axial direction after reaching a threshold (such as a predetermined lower threshold pressure or inflation pressure). In this way, the balloon can be designed to tear in the axial direction before tearing in the lateral direction. As a result, the torn balloon can be more easily retrieved and removed from a patient.

As used herein, the axial direction can refer to a direction that is parallel to a central longitudinal axis of the balloon (e.g., axial direction 124 shown in FIG. 2) and the radial direction can refer to a direction that extends radially outward from the central longitudinal axis of the balloon and is perpendicular to the axial direction. A lateral direction (e.g., lateral direction 126 shown in FIG. 2) can extend perpendicular to the central longitudinal axis of the balloon as well (e.g., across the balloon). A circumferential direction can refer to a direction around a circumference of an object (e.g., the balloon). Further, a thickness of the balloon can be defined in the radial direction, between an inner circumferential surface and an outer circumferential surface of the balloon. When inflated, the inner circumferential surface of the balloon can face and contact an inflation fluid within the balloon.

Designing a balloon to tear in the axial direction can be achieved by varying a durometer of the materials used to create the balloon. For example, the balloon can be extruded to have one or more layers with varying durometer materials. The balloon can then be configured with multiple segments or portions around its circumference (referred to herein as circumferential segments) which have different compliances (or durometers), created by varying layers of the different durometer materials. A higher compliance and/or lower durometer circumferential segment can be configured to rupture (or tear) before a lower compliance and/or higher durometer segment of the balloon.

As used herein, the durometer can refer to the hardness of a material. A higher durometer material can be harder and less compliant than a lower durometer material. Thus, as used herein, a lower durometer material can have a compliance that is higher (more compliant) than a higher durometer material. As an example, since a material with a higher compliance can stretch more than a lower compliance material, the higher compliance material can become thinner as the balloon stretches, thereby causing the material with the lower durometer and higher compliance to tear before the material with the higher durometer and lower compliance.

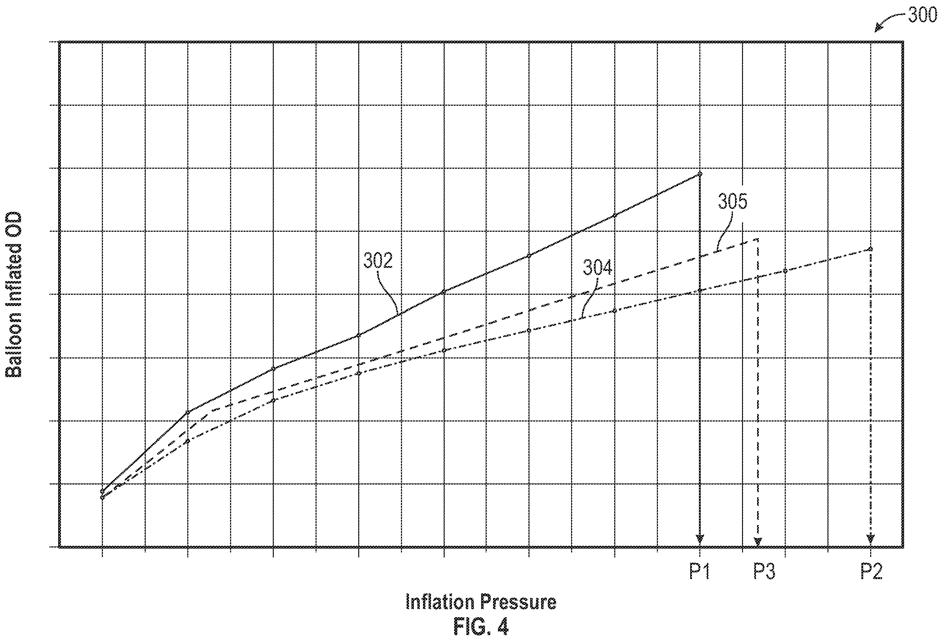

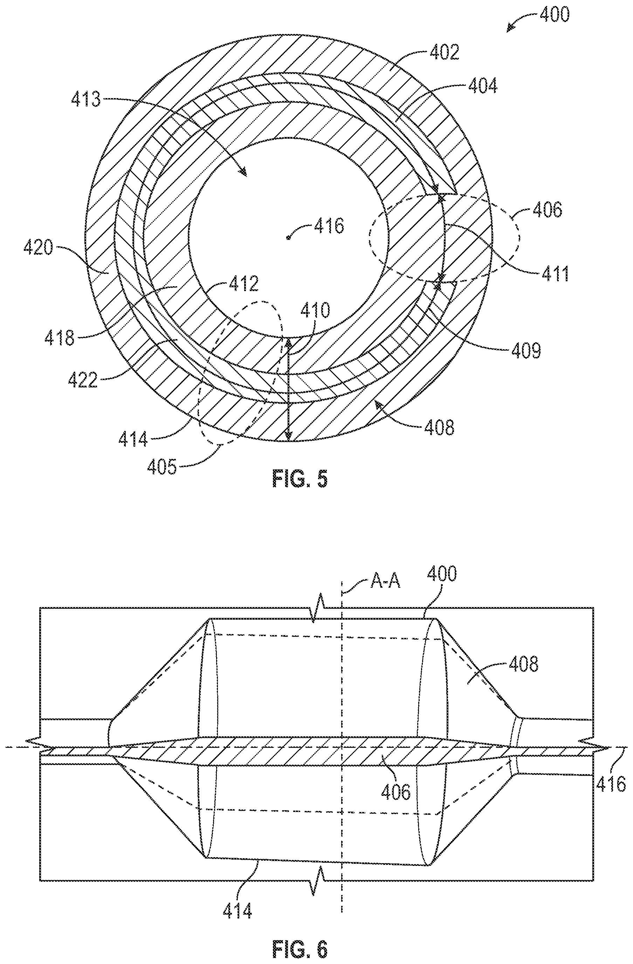

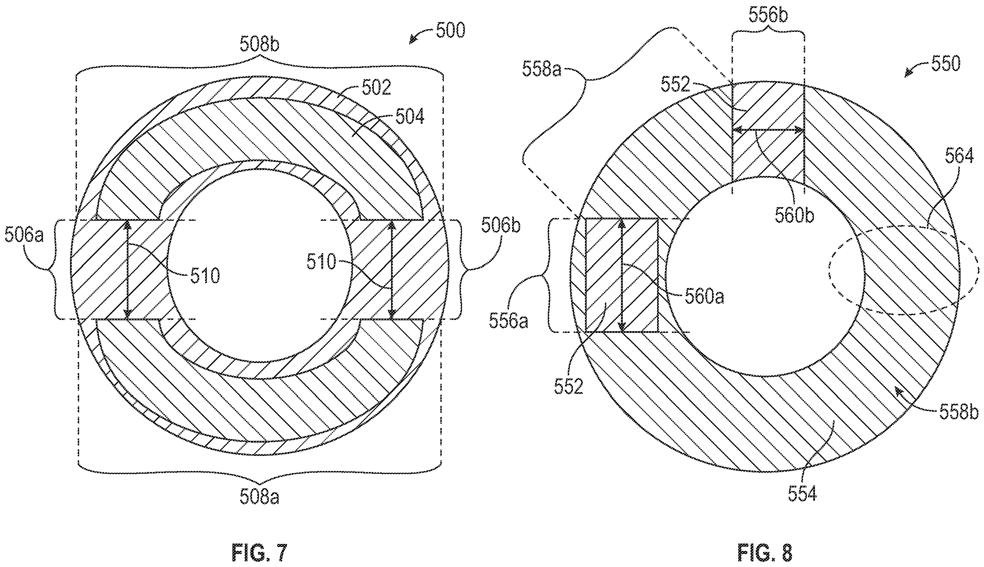

FIG. 4 is a graph 300 illustrating the behavior of different durometer materials within a same balloon or of different segments of the balloon comprising different durometer materials, as the balloon is inflated with an inflation fluid. Specifically, the graph 300 in FIG. 4 shows the outer diameter of the inflated balloon (such as the balloon shown in FIGS. 5 and 6 or in FIG. 8, as described below) on the y-axis and inflation pressure on the x-axis. As the inflation pressure from the inflation fluid within the balloon increases, the outer diameter of the inflated balloon increases. The graph 300 shows three lines or curves that represent the behavior of three different axially-extending circumferential balloon segments of an exemplary balloon. The three lines or curves of graph 300 include a first line 302 showing the outer diameter of a first balloon segment that comprises a first material having a first durometer (lower durometer), a second line 304 showing the outer diameter of a second balloon segment that comprises a second material having a second durometer (higher durometer), the second durometer larger than the first durometer, and a third line 305 showing the outer diameter of a third balloon segment that comprises a combination (e.g., different layer of each) of both the first material and the second material. As shown by first line 302, the first balloon segment that comprises the first material is configured to rupture at a first pressure P1 which is lower than a second pressure P2 at which the second balloon segment comprising the second material is configured to rupture and a third pressure P3 at which the third balloon segment comprising the combination of the first and second materials is configured to rupture. For example, since the first material can have a higher compliance (due to the lower durometer), the first material can stretch more as the inflation pressure increases (and the balloon outer diameter increase), thereby causing it to become thinner and rupture before the second material (since the second material has a lower compliance, stretches less, and thus does not thin as quickly as the first material). Since the third balloon segment comprises a combination of the first and second materials, the third pressure P3 is between the first pressure P1 and the second pressure P2. Whether the third pressure P3 is closer to the first pressure P1 or the second pressure P2 is dependent on whether the proportion of the first material is greater or less than the reproportion of the second material, respectively, in the third balloon segment.

In some examples, the first line 302 can represent the behavior of a first segment 406 in balloon 400 of FIG. 5 and the third line 305 can represent the behavior of a segment 405 in balloon 400 (a smaller section of the second segment 408, which is described further below with reference to FIGS. 5 and 6).

In some examples, the first line 302 can represent the behavior of a first segment 556b in balloon 550 of FIG. 8, the second line 304 can represent the behavior of a segment 564 (a smaller section of the second segment 558b) in balloon 550, and the third line 305 can represent the behavior of a first segment 556a in balloon 550.

FIGS. 5 and 6 show a first exemplary balloon 400 that includes an axially-extending circumferential segment (first segment 406) that is configured to rupture or tear before a remainder of the balloon 400 (e.g., upon the balloon reaching a threshold inflation pressure) such that the balloon 400 ruptures in the axial direction (e.g., along the axially-extending segment). In this way, the balloon 400 can be comprised of relatively stronger and weaker circumferential segments (or relatively more and less resistant to increasing pressure from an inflation fluid) so that the weaker segment ruptures prior to the stronger segment or segments.

The balloon 400 includes a first portion 402 that has a first durometer and a second portion 404 that has a second durometer, the second durometer higher than the first durometer (FIG. 5). As such, the first portion 402 can be more compliant (and thus be configured to stretch more in response to increasing inflation pressure) than the second portion 404. In some examples, the first portion 402 can comprise a first material (the first, lower durometer and higher compliance material of first line 302 in FIG. 4) and the second portion 404 can comprise a second material (the second, higher durometer and lower compliance material of second line 304 in FIG. 4). As described above, the first material can have a lower durometer and a higher compliance than the second material.

FIG. 5 shows a cross-section of the balloon 400 taken along section A-A in FIG. 6. The balloon 400 has an annular cross-section with a wall thickness 410 of the balloon extending between an inner circumferential surface 412 and an outer circumferential surface 414 of the balloon 400. The inner circumferential surface 412 defines a cavity 413 that is configured to receive an inflation fluid when mounted on a shaft of a balloon catheter. In FIG. 5 and the additional cross-sections of the figures described herein, the wall thickness 410 of the balloon relative to the diameter of the cavity and the outer diameter of the balloon as shown may be exaggerated (larger than actual) for the purposes of illustration and clearly showing the different layers of the balloon.

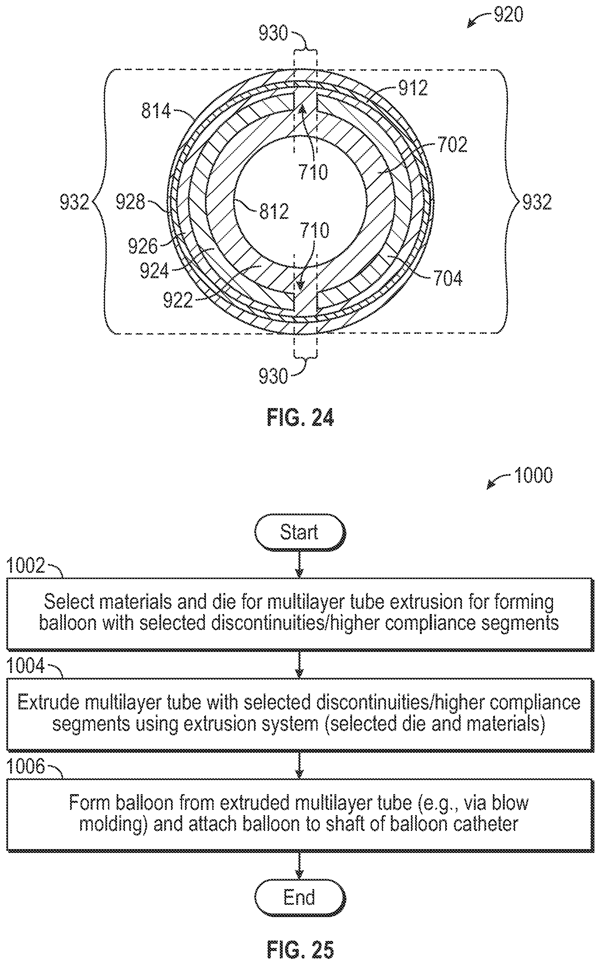

In some examples, the balloon 400 can be a multi-layer balloon where the first portion 402 forms a first layer 418 and a second layer 420 (e.g., of the first material) and the second portion 404 forms a third layer 422 (e.g., of the second material) between the first layer 418 and the second layer 420 (FIG. 5). The different layers of the balloon 400 can be formed using a multi-layer extrusion process as described further below with reference to FIG. 25.

As shown in FIG. 5, the balloon 400 comprises a first segment 406 that extends in a circumferential direction around a portion of a circumference of the balloon 400 and a second segment 408 that extends in the circumferential direction around another portion of the circumference of the balloon (as shown by an arrow which represents an arc length 409 of the second segment 408 in FIG. 5). Thus, the first segment 406 and the second segment 408 can be referred to herein as circumferential segments. In some examples, as shown in FIG. 5, the first segment 406 and the second segment 408 make up an entire circumference of the balloon 400.

As used herein, a "circumferential segment" of the balloon can refer to a segment of the balloon which extends in a circumferential direction around a portion (e.g., only a portion) of a circumference of the balloon. The circumferential segment can extend axially along a length of the balloon and extend in a radial direction across a thickness of the balloon. As such, since a cross-section of the balloon taken by a plane that in normal to the axial direction can be annular, the circumferential segment can form a wedge or section of the annulus.

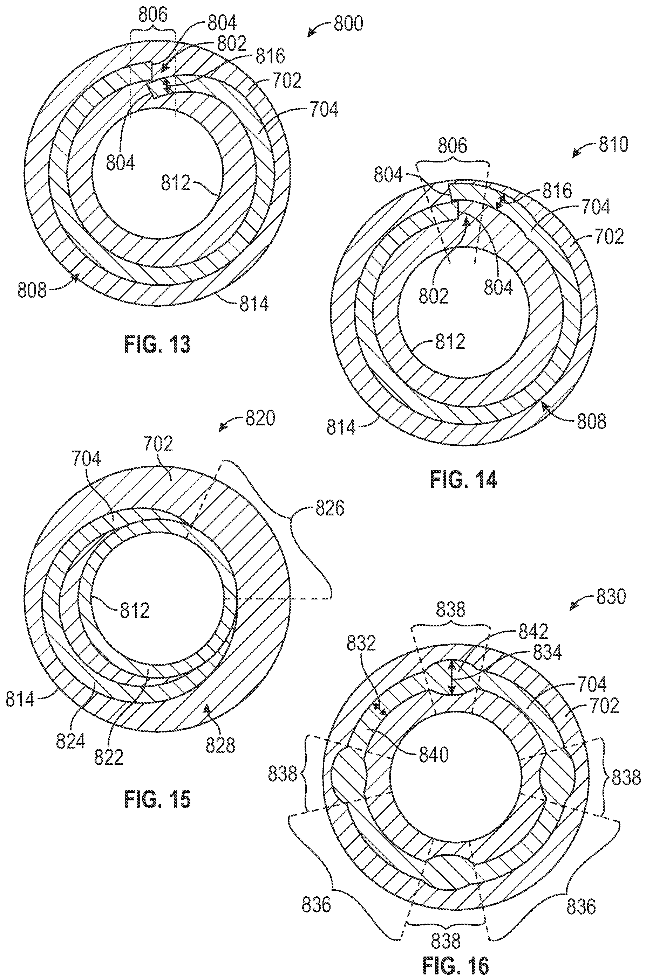

As shown in the example of FIG. 5, the first segment 406 comprises only the first portion 402 (e.g., the lower durometer material). For example, the first segment 406 can comprise one or more layers of the first portion (or first material) and can extend across the entire thickness 410 of the balloon 400, from the inner circumferential surface 412 to the outer circumferential surface 414 of the balloon 400. Thus, in some examples, the first segment 406 can comprise only the lower durometer first material. However, in some examples, the first segment 406 can comprise additional materials (in addition to the first material) and/or discontinuities in the second material or thinner portions of the second material than a remainder of the balloon, as discussed further below with reference to FIGS. 13-25.

The first segment 406 is an axially-extending segment that extends along a length of the balloon 400 (in a direction of a central longitudinal axis 416 of the balloon 400), but around only a portion of the circumference of the balloon 400 (FIG. 6). The second segment 408 can comprise a remainder of the balloon 400 and also extends axially along a length of the balloon (FIG. 6). In the illustrated example, both segments 406, 408 extend axially the entire length of the balloon 400, or at least the entire length of the inflatable portion of the balloon that forms the cavity.

In some examples, the first segment 406 extends axially at least the majority of the length of the balloon.

In some examples, the first segment 406 extends less than the majority of the length of the balloon.

The axial length of each segment formed from the higher compliant material can vary; e.g., each such segment can extend axially less than the majority of the length of the balloon, at least the majority of the length of the balloon, or the entire length of the balloon (or at least the entire length of the inflatable portion of the balloon).

The second segment 408 can span a majority of the circumference of the balloon 400, or for an arc length 409, as shown in FIG. 5. The arc length 409 of the second segment 408 is longer than an arc length 411 of the first segment 406. The second segment 408 can comprise a portion of the first portion 402 (the first layer 418 and the second layer 420) and the second portion 404 (the third layer 422). For example, across the thickness 410, the second segment 408 comprises the third layer 422 of the second portion 404 arranged between the first layer 418 and the second layer 420 of the first portion 402. In this way, the second segment 408 can comprise a band or layer of the second portion 404 (e.g., second material) embedded within the first portion 402 (e.g., first material). Since the second material of the second portion 404 is configured to rupture at a higher pressure than the first material, as shown in the example graph 300 of FIG. 4, the second segment 408 can be stronger or more resistant to rupturing or tearing than the first segment 406.

As a result, the first segment 406 (which does not comprise any of the higher durometer second portion 404 in the example of FIGS. 5 and 6) can be configured to rupture prior to the second segment 408. Since the first segment 406 extends axially along the length of the balloon 400 and extends around only a portion of the circumference of the balloon (FIG. 6), the balloon 400 is configured to rupture in the axial direction along the first segment 406. The second segment 408 arranged on either side of the first segment 406 (in the circumferential direction) can prevent the balloon 400 from tearing laterally across the balloon. In this way, the balloon 400 can be more easily retrieved and removed with the delivery apparatus (or another balloon catheter) when experiencing a rupture.

The first segment 406 of the balloon 400 is configured as a relatively "weaker" circumferential segment (as compared to a remainder of the balloon, or the second segment 408) that is configured to tear at a lower pressure than the remainder of the balloon. Said another way, the first segment 406 can be formed by a discontinuity in the higher durometer second portion 404 (in the circumferential direction). Such a lower durometer (or weaker) circumferential segment (or segments) of the balloon can be created in other ways, by altering an arrangement of the different durometer portions within the balloon. Additional examples of balloons having circumferential segments that are configured to rupture prior to remaining circumferential segments of the balloon (and rupture in the axial direction) are described below with reference to FIGS. 7-25.

FIGS. 7 and 8 show examples of balloons with multiple circumferential segments that are configured to rupture before a remainder of the balloon (and rupture in the axial direction). For example, as shown in FIG. 7, a balloon 500 can comprise a first portion 502 that has a first durometer (and first compliance) and a second portion 504 that has a second durometer (and second compliance), the second durometer greater than the first durometer (similar to as described above for the first portion 402 and second portion 404). In some examples, the first portion 502 can comprise the first material (as described above for the first portion 402 and the second portion 504 can comprise the second material (as described above for the second portion 404).

In some examples, the first portion 502 can comprise a different first material or combination of first materials and the second portion 504 can comprise a different second material or combination of second materials, the second material(s) of the second portion 504 having a higher durometer than the first material(s) of the first portion 502.

The balloon 500 can comprise multiple (two shown in FIG. 7) first segments 506a and 506b (circumferential segments) that comprise the first portion 502 and form discontinuities in the second portion 504, in the circumferential direction. The balloon 500 further comprises multiple second segments 508a and 508b around its circumference that comprise the first portion 502 and the second portion 504. For example, each of the second segments 508a and 508b can comprise a section of the second portion 504 (having the higher, second durometer) embedded within the first portion 502 (having the lower, first durometer). Said another way, each of the second segments 508a and 508b can be formed by an inner and outer layer of the first portion 502 (e.g., first material) and a middle layer of the second portion 504 (e.g., second material).

Similar to the first segment 406 of balloon 400 (FIGS. 5-6), the first segments 506a and 506b can be axially-extending circumferential segments that are configured to rupture (in the axial direction) prior to the second segments 508a and 508b, due to the second segments 508a and 508b including the second portion 504 (which can comprise the higher durometer material).

In some examples, a height 510 (defined in the circumferential direction) of the lower durometer first segments 506a and 506b can be selected based on the materials of the first portion 502 and second portion 504 and/or a desired inflation pressure at which rupturing at the first segments 506a and 506b occurs. For example, the height 510 can be smaller or larger than shown in FIG. 7. Further, in some examples, the heights 510 of the first segments 506a and 506b can be the same or different from one another.

FIG. 8 depicts an exemplary balloon 550 that comprises a first portion 552 that has a first durometer and a second portion 554 that has a second durometer, the second durometer greater than the first durometer (similar to as described above for the first portion 402 and second portion 404). The balloon 550 can comprise a plurality (two shown in FIG. 8) of first segments 556a and 556b around its circumference that comprise the first portion 552 (and in the case of the first segment 556a, also the second portion 554). As an example, the first segment 556a comprises a section or layer of the first portion 552 (having the smaller, first durometer) embedded within the second portion 554 (having the larger, second durometer) and the first segment 556b comprises only one or more layers of the first portion 552 (and none of the second portion 554). In this way, the first segments 556a and 556b can form discontinuities or breaks in the second portion 554 in the circumferential direction of the balloon 550.

The balloon 550 can further comprise a plurality of second segments 558a and 558b, each disposed between the two first segments 556a and 556b. The second segments 558a and 558b can comprise sections of the second portion 554. Thus, the second segments 558a and 558b can comprise a one or more layers of the material of the second portion 554 and the first segments 556a and 556b can comprise either one or more layers of the material of the first portion 552 (first segment 556b) or a layer of the material of the first portion 552 and one or more layers of the material of the second portion 554 (first segment 556a).

Similar to the first segment 406 of balloon 400 (FIGS. 5-6), the first segments 556a and 556b can be axially-extending circumferential segments that are configured to tear (in the axial direction) prior to a remainder of the balloon (the second segments 558a and 558b), due to the first segments 556a and 556b having a higher compliance than the second segments 558a and 558b (from the lower, first durometer of the first portion 552 included in the first segments 556a and 556b). For example, by introducing the lower durometer material of the first portion 552 into the first segments 556a and 556b while a remainder of the balloon comprises the higher durometer material of the second portion 554, the first segments 556a and 556b can be designed to stretch more with increasing inflation pressure and rupture at a lower inflation pressure than the remainder of the balloon 550.

In some examples, the heights 560a and 560b (defined in the circumferential direction) of the lower durometer first segments 556a and 556b, respectively, can be selected based on the materials of the first portion 552 and second portion 554 and/or a desired inflation pressure at which tearing at the first segments 556a and 556b occurs. For example, the heights 560a and 560b can be smaller or larger than shown in FIG. 8. Further, in some examples, the heights 560a and 560b of the first segments 556a and 556b, respectively, can be the same or different from one another.

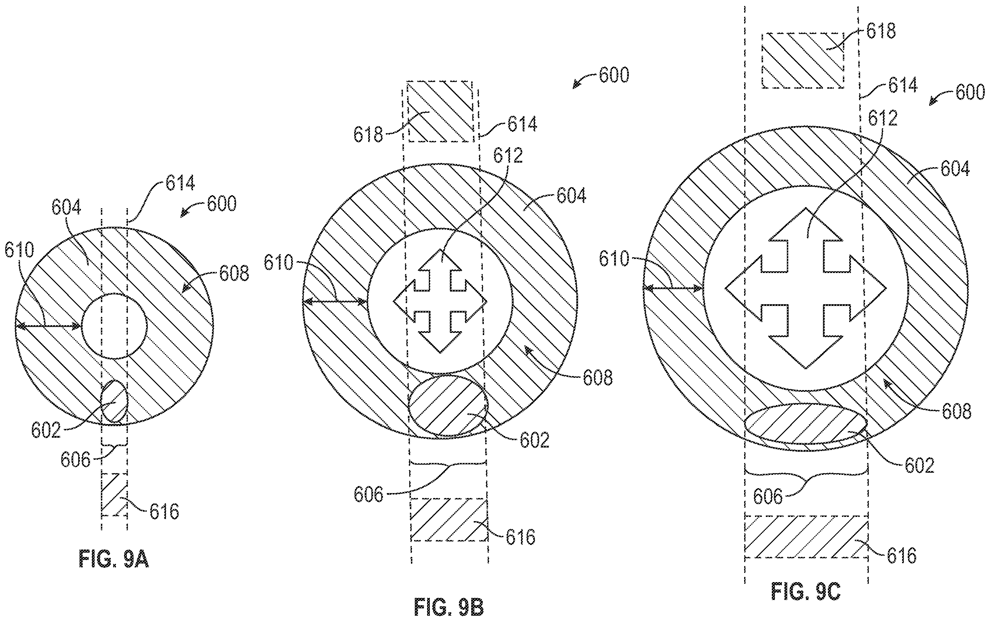

FIGS. 9A-9C show an example of a balloon 600 with an axially-extending circumferential segment of the balloon that is configured to rupture before a remainder of the balloon 600 in the axial direction. The balloon 600 can comprise a first portion 602 with a first durometer and a second portion 604 with a second durometer, the second durometer greater than the first durometer. In some examples, the first portion 602 can comprise a first material having the first durometer (the same or similar to the first portion 402, 502, and/or 552) and the second portion 604 can comprise a second material having the second durometer (the same or similar to the second portion 404, 504, and/or 554).

The balloon 600 can comprise a first circumferential segment 606 that comprises the first portion 602 embedded within the second portion 604. A second circumferential segment 608 can make up a remainder of the balloon 600 and comprise only the second portion 604. The first portion 602 (which can comprise the first material with the lower, first durometer) can either extend partially through a thickness 610 of the wall of the balloon 600 or through the entire thickness 610. In this way, the first portion 602 of the first circumferential segment 606 can be configured to stretch more with increasing inflation pressure, as the balloon 600 is filled with an inflation fluid, than the second portion 604 (FIGS. 9A-9C).

More specifically, FIGS. 9A-9C illustrate how the lower durometer first material of the first portion 602 (which has a higher compliance, as compared to the second portion 604) stretches or expands circumferentially more that the higher durometer material of the second portion 604 as the balloon is inflated with an inflation fluid, thereby causing an increase in inflation pressure. The increasing inflation pressure 612 against the balloon 600 is illustrated by arrows centered within the balloon 600 in FIGS. 9B and 9C.

In FIGS. 9B and 9C, dashed vertical lines 614 and first box 616 schematically illustrate the growth or expansion of the lower durometer first portion 602 in the circumferential direction as the inflation pressure 612 increases and an outer diameter of the balloon 600 increases. Similarly, a second box 618 represents a section of the second portion 604 and schematically illustrates how the second portion 604 grows in size as the inflation pressure 612 increases and the outer diameter of the balloon 600 increases.

As the diameter of the balloon 600 increases under the increasing inflation pressure 612, the first portion 602 of the first circumferential segment 606 grows more than the second portion 604 of the second circumferential segment 608 (e.g., since the first material of the first portion 602 is more compliant and has a lower durometer than the second portion 604). The first portion 602 grows and becomes thinner more quickly with increasing inflation pressure 612 than the remainder of the balloon 600. Thus, the first circumferential segment 606 is configured to rupture first, before the second circumferential segment 608, upon reaching a threshold inflation pressure at which rupture occurs for the material of the first portion 602 (similar to as described above with reference to the graph 300 of FIG. 4).

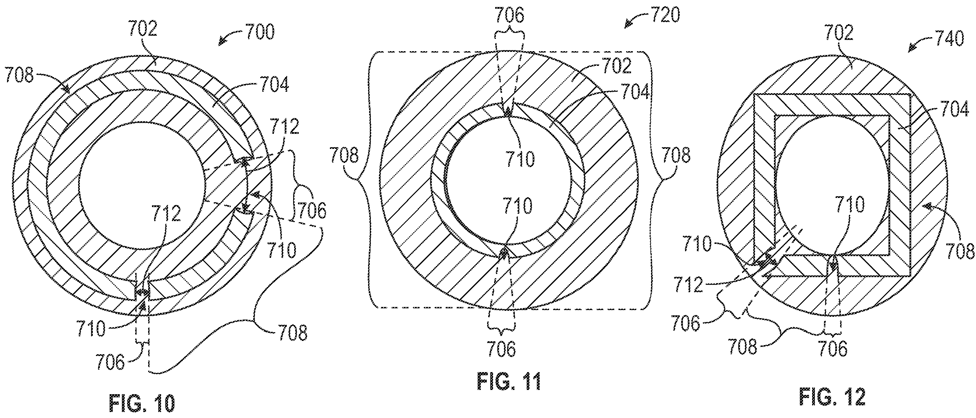

FIGS. 10-12 show cross-sections of exemplary balloons 700, 720, and 740 comprising a first portion 702 with a first durometer and second portion 704 with a second durometer that is larger than the first durometer. In some examples, the first portion 702 can comprise a first material with a lower, first durometer and the second portion 704 can comprise a second material with a higher, second durometer (relative to the first durometer). The first portion 702 can be the same or similar to the other first portions described herein (e.g., the first portion 404) and the second portion 704 can be the same or similar to the second portions described herein (e.g., the second portion 404).

It should be noted that FIGS. 10-24 are all labeled as comprising variously configured (e.g., shaped and sized) first portions 702 and second portions 704 for the sake of simplicity. In these examples, the first portion 702 and second portion 704 can have a lower durometer (and higher compliance) and higher durometer (and lower compliance), respectively, relative to one another. However, between the different balloon examples (in the different FIGS. 10-24), the specific materials that result in the lower durometer first portion 702 and higher durometer second portion 704 can vary.

Returning to FIGS. 10-12, the second portion 704 can be configured as a partial ring or discontinuous layer within or surrounded by the first portion 702. The first portion 702 can be configured as one or more circumferentially extending layers. In this way, the balloons 700, 720, and 740 of FIGS. 10-12 can be multi-layer balloons formed from multiple layer extrusions of different durometer materials (the first portion 702 and the second portion 704).

The balloons 700, 720, and 740 shown in FIGS. 10-12 can have one or more discontinuities 710 in the second portion 704. The discontinuities 710 can be full or partial gaps or breaks in the circumferentially extending second portion 704. These discontinuities 710 can break the continuity of the higher durometer second portion 704 in the circumferential direction, thereby creating relatively weaker first circumferential segments 706 that are configured to rupture under increasing inflation pressure before a remainder of the balloon (e.g., second circumferential segments 708 of the balloon that comprise an unbroken section of the second portion 704). As described herein, the first circumferential segments 706 and the second circumferential segments 708 can extend axially along a length of the balloon.

As one example, the balloon 700 of FIG. 10 includes two discontinuities 710 configured as full gaps or breaks of varying heights 712 (defined in the circumferential direction) in the second portion 704 which create the first circumferential segments 706.

In some examples, the balloon 700 can include more or less than two discontinuities 710 (e.g., only one or three) and they can have the same or different heights 712 as each other.

In this way, the balloon 700 can comprise multiple first circumferential segments 706 which comprise the first portion 702 (e.g., only one or more layers of the material of the first portion 702 across a thickness of the balloon) and that are configured to rupture in the axial direction prior to the second circumferential segments 708.

As shown in FIG. 11, the balloon 720 includes two discontinuities 710 configured as axially extending gaps or notches that extend either partially or fully through the second portion 704 to create the first circumferential segments 706. The discontinuities 710 in the balloon 720 are spaced apart from one another in the circumferential direction.

In some examples, the balloon 720 can include more or less than two discontinuities 710 (e.g., only one or three) and/or the discontinuities 710 can vary in depth (radially into the second portion 704) and/or height 712.

As a result, the balloon 720 comprises multiple first circumferential segments 706 which comprise the first portion 702 and none or only a partial thickness of the second portion 704. As such, the first circumferential segments 706 form a relative weak point in the balloon 720 that is configured to rupture in the axial direction prior to the second circumferential segments 708.

The balloon 740 of FIG. 12 includes two discontinuities 710 configured as full gaps or breaks of varying heights 712 in the second portion 704 which create the first circumferential segments 706.

In some examples, the balloon 740 can include more or less than two discontinuities 710 (e.g., only one or three) and they can have the same or different heights 712 as each other.

In this way, the balloon 700 can comprise multiple first circumferential segments 706 which comprise the first portion 702 (only one or more layers of the material of the first portion 702 across a thickness of the balloon) and that are configured to rupture in the axial direction prior to the second circumferential segments 708. Further, FIG. 12 shows an example of the second portion 704 being configured as a rectangular or square ring rather than the circular ring shown in FIGS. 10 and 11.

In some examples, the ring or layer of material of the second portion 704 can be a different shape that extends circumferentially around the balloon 740, such as a zig-zag pattern, wave pattern, hexagon, or the like.

FIGS. 13-24 depict cross-sections of additional examples of balloons that comprise the first portion 702 having the first durometer (which can comprise the first material or another material or combination of materials having a relatively lower durometer) and the second portion 704 having the second durometer (which can comprise the second material or another material or combination of materials having the relatively higher durometer), where the second durometer is larger than the first durometer. In the different examples of FIGS. 13-24, the shape, arrangement, and relative sizes of the first portion 702 and the second portion 704 within the balloon can vary.

FIGS. 13 and 14 show examples of balloons 800 and 810, respectively, with discontinuities 802 that are created by breaks in the second portion 704 with the longitudinally extending ends 804 of portion 704 overlapping and/or arranged adjacent to one another in a radial direction. These discontinuities 802, which extend axially along a length of the balloon, can result in the formation of a first circumferential segment 806 (illustrated between dashed lines and with a bracket) that is configured to rupture before a remainder of the balloon under increasing inflation pressure (e.g., before a second circumferential segment 808 where the second portion 704 is not broken within the first portion 702). Thus, the first circumferential segment 806 forms a relative "weak" point in the balloon due to a material interruption or discontinuity 802 in the second portion 704 (which has a lower compliance and higher durometer).

In some examples, as shown in the exemplary balloon 800 of FIG. 13, the ends 804 of the second portion 704 of the first circumferential segment 806 can be disposed closer to an inner circumferential surface 812 of the balloon 800.

In some examples, as shown in the exemplary balloon 810 of FIG. 14, the ends 804 of the second portion 704 of the first circumferential segment 806 can be disposed closer to an outer circumferential surface 814 of the balloon 810.

In some examples, the ends 804 of the discontinuity 802 can be approximately centered between the inner circumferential surface 812 and the outer circumferential surface 814 in the radial direction.

In some examples, the ends 804 of the discontinuity 802 can each extend further in the circumferential direction and overlap one another by a greater amount than shown in FIGS. 13 and 14.

In some examples, the ends 804 of the discontinuity 802 can be spaced apart from one another such that a gap is formed in the circumferential direction that is filled entirely by the first portion 702. Further, in some examples, a thickness 816 of the ends 804 of the discontinuity 802 can be smaller than a remainder of the second portion 704.

FIG. 15 shows a cross-section of an exemplary balloon 820 where a compliance or durometer variation in the circumferential direction can be created by altering a number of layers of the second portion 704 within the first portion 702 of the balloon 820. For example, the second portion 704 of the balloon 820 can be formed as two layers, including a first layer 822 and a second layer 824, the second layer 824 arranged radially outward of the first layer 822. In some examples, the first layer 822 can be disposed adjacent to the inner circumferential surface 812 of the balloon 820 (and can form the inner surface 812 as shown in the illustrated example) and the second layer 824 can extend radially outward from a first side of the first layer 822 and toward the outer circumferential surface 814 of the balloon 820. In this manner, the central axis of the second layer 824 can be offset from the central axis of the first layer 822 and the balloon. A first circumferential segment 826 of the balloon 820 can include only the first layer 822 of the second portion 704 and a remainder of the first circumferential segment 826 can be comprised of one or more layers of the first portion 702 (e.g., a plurality of layers of the first portion 702). A second circumferential segment 828 of the balloon 820 can include both the first layer 822 and the second layer 824 of the second portion 704 within the first portion 702 (e.g., the second circumferential segment 828 can be formed by multiple layers of both the first portion 702 and second portion 704, or at least two layers of each of the first portion 702 and the second portion 704). Thus, the first circumferential segment 826 can be relatively weaker and have a higher compliance, thereby causing it to rupture under increasing inflation pressure before the second circumferential segment 828.

FIG. 16 shows a cross-section of an exemplary balloon 830 where a compliance or durometer variation in the circumferential direction can be created by altering a thickness (or amount of material in the radial direction) of the circumferentially extending second portion 704 around the circumference of the balloon 830. For example, the second portion 704 can comprise one or more thinner sections 840 having a first thickness 832 and one or more thicker sections 842 having a second thickness 834. Thus, the balloon 830 can comprise one or more first circumferential segments 836 including a thinner section 840 of the second portion 704 and one or more second circumferential segments 838 including a thicker section 842 of the second portion 704. Thus, the one or more first circumferential segments 836 can have a higher compliance than the one or more second circumferential segments 838 due to the fact that the first circumferential segments 836 include more of the more compliant material of the first portion 702 and less of the less compliant material of the second portion 704 along the radial direction compared to the second circumferential segments 838. As such, the first circumferential segments 836 can be configured to rupture under increasing inflation pressure prior to the one or more second circumferential segments 838.

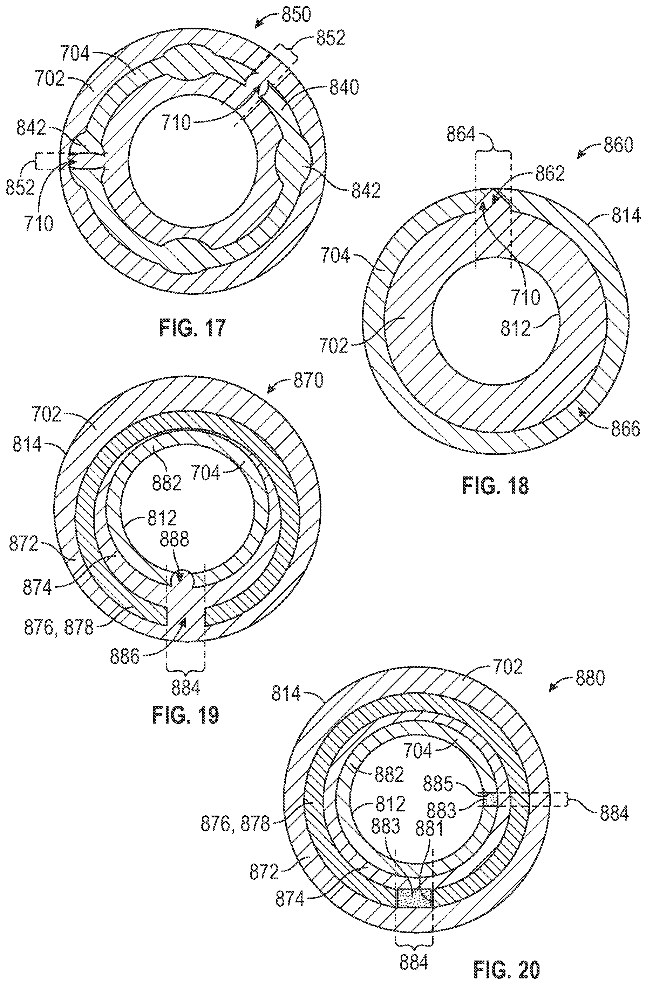

FIG. 17 shows a cross-section of an exemplary balloon 850 that is similar to the balloon 830 of FIG. 16, but the balloon 850 additionally includes one or more discontinuities 710 (or gaps or breaks) in the second portion 704. In some examples, the discontinuity 710 can be in the thicker section 842.

In some examples, the discontinuity 710 can be in the thinner section 840.

In some examples, the discontinuities can have varying heights in the circumferential direction. As discussed above with reference to FIGS. 10-12, these discontinuities 710 can form one or more first segments 852 that comprise the first portion 702 having the smaller durometer (and in some examples none of the second portion 704). Thus, the first segments 852 can be configured to rupture in the axial direction under increasing inflation pressure before a remainder of the balloon 850.

FIG. 18 shows a cross-section of an exemplary balloon 860 where a discontinuity 710 in the second portion 704 is created by a varying height layer 862 of the first portion 702 extending into the second portion 704. In the example of FIG. 18, the second portion 704 is arranged adjacent to the outer circumferential surface 814 of the balloon 860, thereby forming an outermost layer of the balloon 860 while the first portion 702 forms an innermost layer of the balloon 860. FIG. 18 shows an example of how a shape of the discontinuity 710 can vary (e.g., can be triangular instead of rectangular or square). A first circumferential segment 864 that is configured to rupture in the axial direction under increasing inflation pressure prior to a second circumferential segment 866 of the balloon 860 can be formed in a region of the discontinuity 710.

FIGS. 19 and 20 show cross-sections of exemplary balloons 870 and 880, respectively, that comprise a plurality of circumferentially extending layers or portions, including the first portion 702 configured as a first layer 872 and second layer 874, a third portion 876 configured as a third layer 878, and the second portion 704 configured as a fourth layer 882. In some examples, the third portion 876 can have a third durometer that is different (smaller or larger) than the durometers of the first portion 702 and the second portion 704. Further, in some examples, the third portion 876 can comprise a third material (having the third durometer) that is different than the first material of the first portion 702 and the second material of the second portion 704.

The layers of the balloon can either be concentric, as shown in the balloon 880 of FIG. 20, or non-concentric, as shown in the balloon 870 of FIG. 19.

In some examples, one or more first circumferential segments 884 having a higher compliance than a remainder of the balloon can be formed by gaps or discontinuities in one or more layers of the balloon. For example, the balloon 870 of FIG. 19 has a first discontinuity 886 in the third layer 878 and a second discontinuity 888 in the fourth layer 882 which are filled by the first portion 702.

In some examples, the discontinuities can be filled by an additional material or portion that has a lower durometer and higher compliance relative to the layer in which the discontinuity is located, thereby forming the first circumferential segments 884. For example, the balloon 880 of FIG. 20 can have a first discontinuity 881 in the third layer 878 filled by a fourth portion 883 (or fourth material) and a second discontinuity 885 in the fourth layer 882 filled by the fourth portion 883. In some examples, the material filling the first discontinuity 881 and the second discontinuity 885 can be different materials than each other with durometers that are smaller than a durometer of the layers in which they are located.

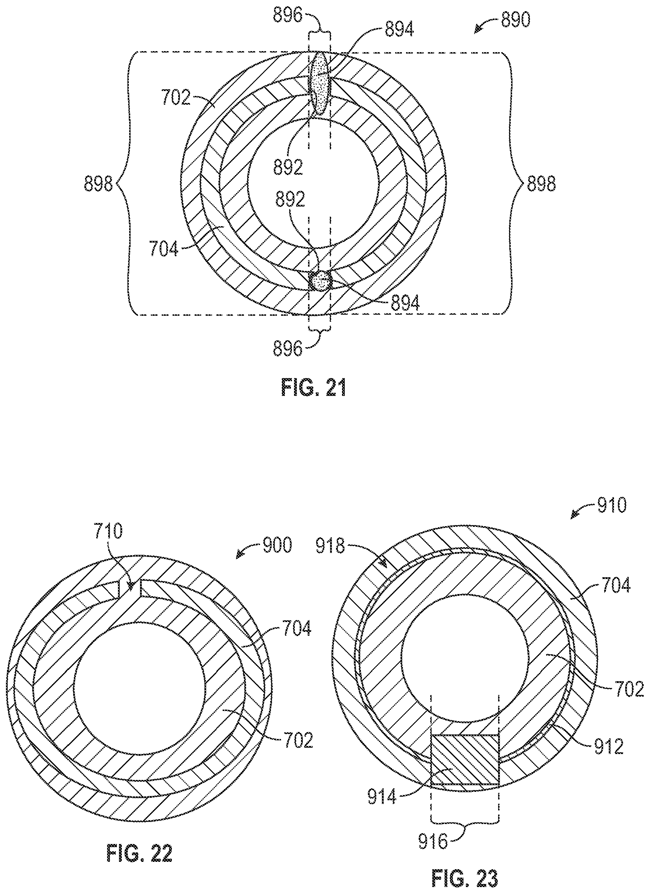

FIG. 21 shows a cross-section of an exemplary balloon 890 including one or more discontinuities 892 (two shown in FIG. 21) in the second portion 704 that are filled with an additional, third portion 894. The third portion 894 can comprise a third material that has a third durometer. In some examples, the third durometer is lower than the second durometer of the second portion 704. In some examples, the third durometer can be lower than the first durometer of the first portion 702 and the second durometer of the second portion 704.

In some examples, the third durometer can be higher than the first durometer of the first portion 702 and lower the second durometer of the second portion 704.

In this way, first circumferential segments 896 that are configured to rupture in the axial direction before a remainder (or remaining second circumferential segments 898) of the balloon 890 can be created in regions of the third portion 894.

In some examples, the third portion 894 can fill only an area of the discontinuity 892 in the second portion 704 (as shown in the bottom portion of FIG. 21). In some examples, the third portion 894 can fill the area of the discontinuity 892 and further extend out of the discontinuity toward the inner circumferential surface 812 and/or the outer circumferential surface 814 (as shown in the top portion of FIG. 21).

FIG. 22 shows a cross-section of an exemplary balloon 900 that is similar to the balloon 400 of FIGS. 5 and 6 and the balloon 700 of FIG. 10; however, instead of a completely circular, annular second portion 704, the second portion 704 is an oblong or oval-shaped layer. Further, as shown in FIG. 22, the second portion 704 can have one or more discontinuities 710 that are filled by the first portion 702. In this way, FIG. 22 shows an example of the balloon 900 where one or more circumferentially extending layers of the balloon (e.g., second portion 704) are not circular, but have a different shape (also shown in the balloon 740 of FIG. 12).

FIG. 23 shows a cross-section of an exemplary balloon 910 that includes a third portion 912 (or portion) between the first portion 702 (first layer) and the second portion 704 (second layer). In some examples, the third portion 912 can be configured as a tie layer that can help support or secure the layers of the first portion 702 and second portion 704 to one another. For example, the third portion 912 can comprise an additional, third material (different than the materials of the first portion 702 and the second portion 704) that help secure or tie the layers of the first portion 702 and second portion 704 to one another.

Additionally, in some examples, the balloon 910 can include a discontinuity (gap or break) in each of the first portion 702, second portion 704, and the third portion 912 that is filled by a fourth portion 914. The fourth portion 914 can comprise a fourth material that is different than the first material of the first portion 702 and the second material of the second portion 704. In some examples, the fourth material of the fourth portion 914 can have a lower durometer than the second portion 704. In some examples, the fourth material of the fourth portion 914 can have a lower durometer than the second portion 704 and the first portion 702. In this way, a first circumferential segment 916 that has a higher compliance than a remainder of the balloon 910 and is configured to rupture in the axial direction before a second circumferential section 918 of the balloon 900 is formed in a region of the fourth portion 914.

In some examples, the third portion 912 can comprise a layer of the third material that is spaced radially away from the second portion 704 and disposed within the first portion 702, as shown in the exemplary balloon 920 of FIG. 24. For example, the balloon 920 can comprise (from the inner circumferential surface 812 to the outer circumferential surface 814) a first layer 922 of the first portion 702, a layer 924 of the second portion 704, a second layer 926 of the first portion 702, a layer of the third portion 912, and finally a third layer 928 of the first portion 702.

Additionally, the balloon 920 can comprise one or more discontinuities 710 filed by the first portion 702 (FIG. 24). As such, first circumferential segments 930 that have a higher compliance than a remainder of the balloon 920 and are configured to rupture in the axial direction before second circumferential sections 932 of the balloon 920 are formed in regions of the discontinuities 710.

FIG. 25 is flow chart of a method 1000 for forming a balloon with one or more circumferential segments that have a higher compliance (and/or lower durometer) than a remaining portion of the balloon such that the one or more circumferential segments are configured to rupture in an axial direction before a remainder of the balloon, when the balloon is under pressure from an inflation fluid introduced into the balloon. For example, the method 1000 can be used to form any of the balloons described herein. In some examples, the balloons formed with method 1000 are multilayer balloons comprising multiple circumferentially extending portions or layers that vary in durometer. In some examples, a higher durometer (and lower compliance) portion or layer of the balloon can comprise one or more discontinuities therein that are filled with a lower compliance portion or material, thereby forming the one or more higher compliance circumferential segments that are configured to rupture at a lower inflation pressure than a remainder of the balloon.

The method 1000 begins as 1002 and includes selecting one or more materials and a die for the multilayer tube extrusion for forming a balloon with one or more selected discontinuities and/or higher compliance segments (e.g., first segment 406 of balloon 400 of FIGS. 5 and 6). As discussed above, the various portions of the balloon described herein can have different compliances and durometers that are formed by different durometer materials. As an example, such as for the balloon 400 of FIGS. 5 and 6, a first material having a first durometer (for first portion 402) can be selected and a second material having a second durometer (for second portion 404) can be selected, where the second durometer is greater than the first durometer. A die used in the tube extrusion process can be selected that is configured to form the specified layers (portions) and discontinuities (or higher compliance circumferential segments) of the selected balloon.

At 1004, the method 1000 includes extruding a multilayer tube with the selected discontinuities and/or higher compliance circumferential segments using an extrusion system, including the selected die and materials. In some examples, the extrusion system can include a multi (e.g., two or more) material co-extrusion head and a die with a plurality of flow channels configured to form the specified layers of the selected balloon. Using the extrusion system, the selected multilayer tube is formed, which may have a cross-section similar to one of the balloons described herein with reference to FIGS. 5-24.

At 1006, the method 1000 includes forming the balloon from the extruded multilayer tube (e.g., via blow molding) and attaching the formed balloon to a shaft of a balloon catheter. In some examples, the balloon catheter can be a delivery apparatus for a radially expandable prosthetic medical device, such as the delivery apparatus shown in FIG. 2.

In some examples, the higher compliance and/or lower durometer axially extending circumferential segments that are configured to rupture in the axial direction along the balloon prior to a remainder (or lower compliance and/or higher durometer circumferential segments) of the balloon can be formed by varying a wall thickness of the balloon.

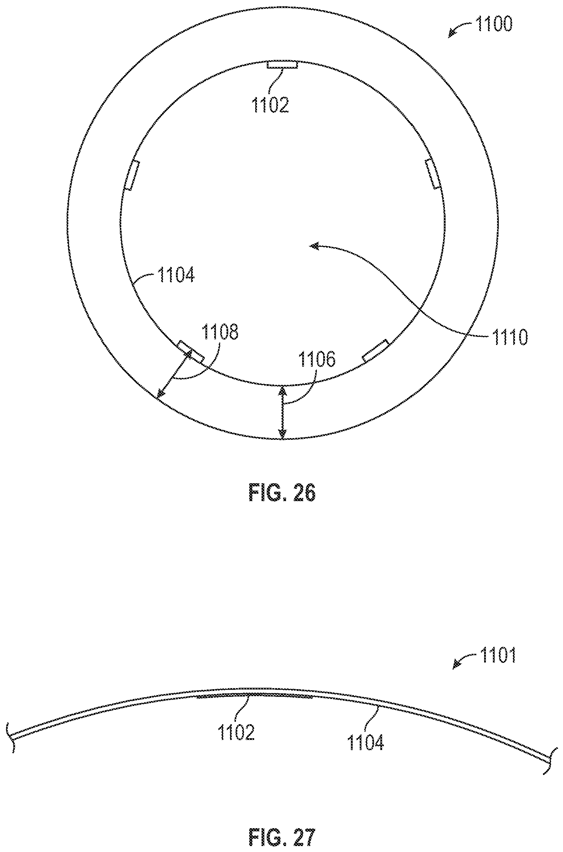

In some examples, as shown in FIGS. 26 and 27, one of more reinforcement elements 1102 can be disposed on an inner circumferential surface 1104 of an extruded balloon tube 1100 (FIG. 26) and a balloon 1101 formed from the balloon tube 1100 (FIG. 27).

In some examples, the one or more reinforcement elements 1102 can be disposed on an outer circumferential surface of the extruded balloon tube 1100 and the balloon 1101 formed from the balloon tube 1100.

In some examples, the one or more reinforcement elements 1102 can be disposed on both the inner circumferential surface 1104 and the outer circumferential surface of the extruded balloon tube 1100 and the balloon 1101 formed from the balloon tube 1100.

Each reinforcement element 1102 extends in an axial direction (into the page in FIGS. 26 and 27) along the balloon tube 1100 and balloon 1101. The one or more reinforcement elements 1102 can add thickness to the balloon tube 1100, and thus the formed balloon 1101, in a radial direction, at a selected circumferential position along an axial length of the balloon. For example, as shown in FIG. 26 (which shows a cross-sectional view of the balloon tube 1100 prior to forming into the balloon 1101) the balloon tube 1100 has a first thickness 1106 at segments of the balloon tube that do not include the reinforcement elements 1102. Further, at segments of the balloon tube that include the reinforcement elements 1102, the balloon tube 1100 has a second thickness 1108 that is greater than the first thickness 1106. The segments of the balloon that do not include the reinforcement elements 1102 can have a higher compliance than the segments of the balloon that include the reinforcement elements.

Thus, the reinforcement elements 1102 can cause the balloon 1101 to rupture in the axial direction along a length of the balloon, instead of laterally (across the balloon). For example, the reinforcement elements 1102 can prevent the balloon from rupturing laterally across the balloon under increasing pressure from an inflation fluid introduced into an interior cavity 1110 of the balloon.

In some examples, the reinforcement elements 1102 can be configured as ribs that extend radially away from the inner surface 1104 and inward toward a central longitudinal axis of the balloon tube 1100 and balloon 1101.

FIG. 27 shows a cross-section of a portion of the formed balloon 1101 (formed from the balloon tube 1100). In some examples, the balloon 1101 can comprise a first material and the reinforcement elements 1102 can comprise a second material that is different than the first material. In some examples, the second material can comprise a higher durometer material than the first material. In some examples, the balloon 1101 can comprise a first material and the reinforcement elements 1102 can comprise the same first material.

In some examples, as shown in FIG. 26, the balloon tube 1100, and thus the balloon 1101, can comprise a plurality of reinforcement element 1102 that are spaced apart from one another in the circumferential direction, around the inner circumferential surface 1104. The spacing between adjacent reinforcement elements can be the same or irregular.

In some examples, each reinforcement element 1102 can extend axially along an entire length of the balloon 1101.

In some examples, the reinforcement elements 1102 can have varying lengths with one or more extending axially along at least a majority of the length of the balloon 1101.

The balloons described herein can be used in various medical catheters that are configured to mount the balloon on a distal end portion of the medical catheter and inflate the balloon (with an inflation fluid) during a medical procedure (and thus can also be referred to as balloon catheters). Examples of such balloon catheters include a delivery apparatus for a radially expandable prosthetic medical device (such as the delivery apparatus 100 of FIG. 2), an angioplasty balloon catheter, and the like. Balloon catheters that include balloons disclosed herein can be used to implant any of various medical devices (e.g., prosthetic heart valves, stents, stent grafts, etc.) or can be used to perform other medical procedures that do not involve implanting a medical device, such as a valvuloplasty procedure.

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) is introduced into the aorta through a surgical incision in the ascending aorta, such as through a partial J-sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

**In** all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

Any of the systems, devices, apparatuses, etc. herein can be sterilized (for example, with heat/thermal, pressure, steam, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

In view of the many possible ways in which the principles of the disclosure may be applied, it should be recognized that the illustrated configurations depict examples of the disclosed technology and should not be taken as limiting the scope of the disclosure nor the claims. Rather, the scope of the claimed subject matter is defined by the following claims.

## Claims

1. An inflatable balloon (108; 400; 500; 550; 600; 700; 720; 740; 800; 810; 820; 830; 850; 860; 870; 880; 890; 900; 910; 920; 1101) for a medical catheter (100), comprising:
a first segment (406; 506a, 506b; 556a, 556b; 606; 706; 806; 826; 836; 852; 864; 884; 896; 916; 930) that has a first compliance; and
a second segment (408; 508a, 508b; 558a, 558b; 608; 708; 808; 828; 838; 866; 898; 918; 932) that has a second compliance, the first compliance higher than the second compliance, wherein the first segment (406; 506a, 506b; 556a, 556b; 606; 706; 806; 826; 836; 852; 864; 884; 896; 916; 930) and the second segment (408; 508a, 508b; 558a, 558b; 608; 708; 808; 828; 838; 866; 898; 918; 932) extend axially along a length of the balloon (108; 400; 500; 550; 600; 700; 720; 740; 800; 810; 820; 830; 850; 860; 870; 880; 890; 900; 910; 920; 1101) and are arranged adjacent to each other in a circumferential direction of the balloon (108; 400; 500; 550; 600; 700; 720; 740; 800; 810; 820; 830; 850; 860; 870; 880; 890; 900; 910; 920; 1101), and wherein the first segment (406; 506a, 506b; 556a, 556b; 606; 706; 806; 826; 836; 852; 864; 884; 896; 916; 930) is configured to rupture prior to the second segment (408; 508a, 508b; 558a, 558b; 608; 708; 808; 828; 838; 866; 898; 918; 932) in an axial direction (124) under pressure (612) from an inflation fluid introduced into the balloon (108; 400; 500; 550; 600; 700; 720; 740; 800; 810; 820; 830; 850; 860; 870; 880; 890; 900; 910; 920; 1101).

2. The balloon (108; 400; 500; 550; 600; 700; 720; 740; 800; 810; 820; 830; 850; 860; 870; 880; 890; 900; 910; 920; 1101) of claim 1, wherein the first segment (406; 506a, 506b; 556a, 556b; 606; 706; 806; 826; 836; 852; 864; 884; 896; 916; 930) and the second segment (408; 508a, 508b; 558a, 558b; 608; 708; 808; 828; 838; 866; 898; 918; 932) are circumferential segments of the balloon (108; 400; 500; 550; 600; 700; 720; 740; 800; 810; 820; 830; 850; 860; 870; 880; 890; 900; 910; 920; 1101) that each extend in a circumferential direction around different portions of a circumference of the balloon (108; 400; 500; 550; 600; 700; 720; 740; 800; 810; 820; 830; 850; 860; 870; 880; 890; 900; 910; 920; 1101).

3. The balloon (108; 400; 500; 550; 600; 700; 720; 740; 800; 810; 820; 830; 850; 860; 870; 880; 890; 900; 910; 920; 1101) of either claim 1 or claim 2, wherein the first segment (406; 506a, 506b; 556a, 556b; 606; 706; 806; 826; 836; 852; 864; 884; 896; 916; 930) comprises a first material having the first compliance and a first durometer, and wherein the second segment (408; 508a, 508b; 558a, 558b; 608; 708; 808; 828; 838; 866; 898; 918; 932) comprises the first material and a second material having a second durometer that is greater than the first durometer.

4. The balloon (108; 400; 500; 550; 600; 700; 720; 740; 800; 810; 820; 830; 850; 860; 870; 880; 890; 900; 910; 920; 1101) of claim 3, wherein the first segment (406; 506a, 506b; 556a, 556b; 606; 706; 806; 826; 836; 852; 864; 884; 896; 916; 930) comprises one or more circumferentially extending layers (418, 420) of the first material which extend across a thickness of the balloon (108; 400; 500; 550; 600; 700; 720; 740; 800; 810; 820; 830; 850; 860; 870; 880; 890; 900; 910; 920; 1101), and wherein the second segment (408; 508a, 508b; 558a, 558b; 608; 708; 808; 828; 838; 866; 898; 918; 932) comprises one or more circumferentially extending layers (418, 420) of the first material and one or more circumferentially extending layers (422) of the second material.

5. The balloon (108; 400; 500; 550; 600; 700; 720; 740; 800; 810; 820; 830; 850; 860; 870; 880; 890; 900; 910; 920; 1101) of claim 3, wherein the first segment (406; 506a, 506b; 556a, 556b; 606; 706; 806; 826; 836; 852; 864; 884; 896; 916; 930) comprises a plurality of circumferentially extending layers (418, 420) of only the first material, and wherein the second segment (408; 508a, 508b; 558a, 558b; 608; 708; 808; 828; 838; 866; 898; 918; 932) comprises one or more circumferentially extending layers (418, 420) of the first material and one or more circumferentially extending layers (422) of the second material

6. The balloon (108; 400; 500; 550; 600; 700; 720; 740; 800; 810; 820; 830; 850; 860; 870; 880; 890; 900; 910; 920; 1101) of claim 3, wherein the first segment (406; 506a, 506b; 556a, 556b; 606; 706; 806; 826; 836; 852; 864; 884; 896; 916; 930) comprises a plurality of circumferentially extending layers (418, 420) of the first material and a single circumferentially extending layer (422) of the second material, and wherein the second segment (408; 508a, 508b; 558a, 558b; 608; 708; 808; 828; 838; 866; 898; 918; 932) comprises a plurality of circumferentially extending layers (418, 420) of the first material and at least two circumferentially extending layers (422) of the second material.

7. The balloon (108; 400; 500; 550; 600; 700; 720; 740; 800; 810; 820; 830; 850; 860; 870; 880; 890; 900; 910; 920; 1101) of claim 4, wherein the second segment (408; 508a, 508b; 558a, 558b; 608; 708; 808; 828; 838; 866; 898; 918; 932) comprises one circumferentially extending layer (422) of the second material disposed between multiple circumferentially extending layers (418, 420) of the first material.

8. The balloon (108; 400; 500; 550; 600; 700; 720; 740; 800; 810; 820; 830; 850; 860; 870; 880; 890; 900; 910; 920; 1101) of claim 3, wherein the first segment (406; 506a, 506b; 556a, 556b; 606; 706; 806; 826; 836; 852; 864; 884; 896; 916; 930) comprises more circumferentially extending layers (418, 420) of the first material than the second material, and wherein the second segment (408; 508a, 508b; 558a, 558b; 608; 708; 808; 828; 838; 866; 898; 918; 932) comprises more circumferentially extending layers (422) of the second material than the first segment (406; 506a, 506b; 556a, 556b; 606; 706; 806; 826; 836; 852; 864; 884; 896; 916; 930).

9. The balloon (108; 400; 500; 550; 600; 700; 720; 740; 800; 810; 820; 830; 850; 860; 870; 880; 890; 900; 910; 920; 1101) of any one of claims 1 to 8, wherein the balloon comprises a plurality of first segments (406; 506a, 506b; 556a, 556b; 606; 706; 806; 826; 836; 852; 864; 884; 896; 916; 930) having the first compliance and a plurality of second segments (408; 508a, 508b; 558a, 558b; 608; 708; 808; 828; 838; 866; 898; 918; 932) having the second compliance, and wherein each first segment (406; 506a, 506b; 556a, 556b; 606; 706; 806; 826; 836; 852; 864; 884; 896; 916; 930) is disposed between two adjacent second segments (408; 508a, 508b; 558a, 558b; 608; 708; 808; 828; 838; 866; 898; 918; 932) in a circumferential direction of the balloon.

10. The balloon (108; 400; 500; 550; 600; 700; 720; 740; 800; 810; 820; 830; 850; 860; 870; 880; 890; 900; 910; 920; 1101) of either claim 1 or claim 2, wherein the first segment (406; 506a, 506b; 556a, 556b; 606; 706; 806; 826; 836; 852; 864; 884; 896; 916; 930) comprises a first material having a first durometer embedded within a second material having a second durometer that is greater than the first durometer, and wherein the second segment (408; 508a, 508b; 558a, 558b; 608; 708; 808; 828; 838; 866; 898; 918; 932) comprises the second material.

11. The balloon (108; 400; 500; 550; 600; 700; 720; 740; 800; 810; 820; 830; 850; 860; 870; 880; 890; 900; 910; 920; 1101) of either claim 1 or claim 2, wherein the balloon comprises a first circumferentially extending portion comprising a first material having a first durometer and a second circumferentially extending portion comprising a second material having a second durometer, the second durometer greater than the first durometer, and wherein the first segment (406; 506a, 506b; 556a, 556b; 606; 706; 806; 826; 836; 852; 864; 884; 896; 916; 930) is formed by a break in the second circumferentially extending portion such that a discontinuity (710; 802; 881; 885; 886; 888; 892) is formed in a circumferential direction.

12. The balloon (108; 400; 500; 550; 600; 700; 720; 740; 800; 810; 820; 830; 850; 860; 870; 880; 890; 900; 910; 920; 1101) of any one of claims 1 to 11, wherein the medical catheter is a delivery apparatus for a radially expandable medical device.

13. The balloon (108; 400; 500; 550; 600; 700; 720; 740; 800; 810; 820; 830; 850; 860; 870; 880; 890; 900; 910; 920; 1101) of any one of claims 1 to 12, wherein an arc length of the second segment (408; 508a, 508b; 558a, 558b; 608; 708; 808; 828; 838; 866; 898; 918; 932) is longer than an arc length of the first segment (406; 506a, 506b; 556a, 556b; 606; 706; 806; 826; 836; 852; 864; 884; 896; 916; 930).

14. The balloon (108; 400; 500; 550; 600; 700; 720; 740; 800; 810; 820; 830; 850; 860; 870; 880; 890; 900; 910; 920; 1101) of any one of claims 1 to 13, wherein the medical catheter (100) is a delivery apparatus for a radially expandable medical device, and wherein the balloon (108; 400; 500; 550; 600; 700; 720; 740; 800; 810; 820; 830; 850; 860; 870; 880; 890; 900; 910; 920; 1101) is configured to be attached to a shaft (104, 106) of the delivery apparatus.

15. A balloon catheter (100) comprising:
a shaft (104, 106) extending from a handle (102) of the balloon catheter; and
the inflatable balloon (108; 400; 500; 550; 600; 700; 720; 740; 800; 810; 820; 830; 850; 860; 870; 880; 890; 900; 910; 920; 1101) according to at least one of the preceding claims mounted to the shaft (104, 106).

## Patentansprüche

1. Aufblasbarer Ballon (108; 400; 500; 550; 600; 700; 720; 740; 800; 810; 820; 830; 850; 860; 870; 880; 890; 900; 910; 920; 1101) für einen medizinischen Katheter (100), umfassend:
ein erstes Segment (406; 506a, 506b; 556a, 556b; 606; 706; 806; 826; 836; 852; 864; 884; 896; 916; 930), das eine erste Nachgiebigkeit aufweist; und
ein zweites Segment (408; 508a, 508b; 558a, 558b; 608; 708; 808; 828; 838; 866; 898; 918; 932), das eine zweite Nachgiebigkeit aufweist, wobei die erste Nachgiebigkeit höher ist als die zweite Nachgiebigkeit, wobei sich das erste Segment (406; 506a, 506b; 556a, 556b; 606; 706; 806; 826; 836; 852; 864; 884; 896; 916; 930) und das zweite Segment (408; 508a, 508b; 558a, 558b; 608; 708; 808; 828; 838; 866; 898; 918; 932) axial entlang einer Länge des Ballons (108; 400; 500; 550; 600; 700; 720; 740; 800; 810; 820; 830; 850; 860; 870; 880; 890; 900; 910; 920; 1101) erstrecken und in einer Umfangsrichtung des Ballons (108; 400; 500; 550; 600; 700; 720; 740; 800; 810; 820; 830; 850; 860; 870; 880; 890; 900; 910; 920; 1101) benachbart zueinander angeordnet sind, und wobei das erste Segment (406; 506a, 506b; 556a, 556b; 606; 706; 806; 826; 836; 852; 864; 884; 896; 916; 930) dazu ausgelegt ist, vor dem zweiten Segment (408; 508a, 508b; 558a, 558b; 608; 708; 808; 828; 838; 866; 898; 918; 932) in einer axialen Richtung (124) unter Druck (612) von einem in den Ballon (108; 400; 500; 550; 600; 700; 720; 740; 800; 810; 820; 830; 850; 860; 870; 880; 890; 900; 910; 920; 1101) eingebrachten Inflationsfluid zu bersten.

2. Ballon (108; 400; 500; 550; 600; 700; 720; 740; 800; 810; 820; 830; 850; 860; 870; 880; 890; 900; 910; 920; 1101) gemäß Anspruch 1, wobei das erste Segment (406; 506a, 506b; 556a, 556b; 606; 706; 806; 826; 836; 852; 864; 884; 896; 916; 930) und das zweite Segment (408; 508a, 508b; 558a, 558b; 608; 708; 808; 828; 838; 866; 898; 918; 932) Umfangssegmente des Ballons (108; 400; 500; 550; 600; 700; 720; 740; 800; 810; 820; 830; 850; 860; 870; 880; 890; 900; 910; 920; 1101) sind, die sich jeweils in einer Umfangsrichtung um unterschiedliche Abschnitte eines Umfangs des Ballons (108; 400; 500; 550; 600; 700; 720; 740; 800; 810; 820; 830; 850; 860; 870; 880; 890; 900; 910; 920; 1101) erstrecken.

3. Ballon (108; 400; 500; 550; 600; 700; 720; 740; 800; 810; 820; 830; 850; 860; 870; 880; 890; 900; 910; 920; 1101) gemäß Anspruch 1 oder Anspruch 2, wobei das erste Segment (406; 506a, 506b; 556a, 556b; 606; 706; 806; 826; 836; 852; 864; 884; 896; 916; 930) ein erstes Material mit der ersten Nachgiebigkeit und einem ersten Härtegrad umfasst, und wobei das zweite Segment (408; 508a, 508b; 558a, 558b; 608; 708; 808; 828; 838; 866; 898; 918; 932) das erste Material und ein zweites Material mit einem zweiten Härtegrad umfasst, der größer ist als der erste Härtegrad.

4. Ballon (108; 400; 500; 550; 600; 700; 720; 740; 800; 810; 820; 830; 850; 860; 870; 880; 890; 900; 910; 920; 1101) gemäß Anspruch 3, wobei das erste Segment (406; 506a, 506b; 556a, 556b; 606; 706; 806; 826; 836; 852; 864; 884; 896; 916; 930) eine oder mehrere sich in Umfangsrichtung erstreckende Schichten (418, 420) des ersten Materials umfasst, die sich über eine Dicke des Ballons (108; 400; 500; 550; 600; 700; 720; 740; 800; 810; 820; 830; 850; 860; 870; 880; 890; 900; 910; 920; 1101) erstrecken, und wobei das zweite Segment (408; 508a, 508b; 558a, 558b; 608; 708; 808; 828; 838; 866; 898; 918; 932) eine oder mehrere sich in Umfangsrichtung erstreckende Schichten (418, 420) des ersten Materials und eine oder mehrere sich in Umfangsrichtung erstreckende Schichten (422) des zweiten Materials umfasst.

5. Ballon (108; 400; 500; 550; 600; 700; 720; 740; 800; 810; 820; 830; 850; 860; 870; 880; 890; 900; 910; 920; 1101) gemäß Anspruch 3, wobei das erste Segment (406; 506a, 506b; 556a, 556b; 606; 706; 806; 826; 836; 852; 864; 884; 896; 916; 930) eine Mehrzahl von sich in Umfangsrichtung erstreckenden Schichten (418, 420) ausschließlich des ersten Materials umfasst, und wobei das zweite Segment (408; 508a, 508b; 558a, 558b; 608; 708; 808; 828; 838; 866; 898; 918; 932) eine oder mehrere sich in Umfangsrichtung erstreckende Schichten (418, 420) des ersten Materials und eine oder mehrere sich in Umfangsrichtung erstreckende Schichten (422) des zweiten Materials umfasst.

6. Ballon (108; 400; 500; 550; 600; 700; 720; 740; 800; 810; 820; 830; 850; 860; 870; 880; 890; 900; 910; 920; 1101) gemäß Anspruch 3, wobei das erste Segment (406; 506a, 506b; 556a, 556b; 606; 706; 806; 826; 836; 852; 864; 884; 896; 916; 930) eine Mehrzahl von sich in Umfangsrichtung erstreckenden Schichten (418, 420) des ersten Materials und eine einzelne sich in Umfangsrichtung erstreckende Schicht (422) des zweiten Materials umfasst, und wobei das zweite Segment (408; 508a, 508b; 558a, 558b; 608; 708; 808; 828; 838; 866; 898; 918; 932) eine Mehrzahl von sich in Umfangsrichtung erstreckenden Schichten (418, 420) des ersten Materials und mindestens zwei sich in Umfangsrichtung erstreckende Schichten (422) des zweiten Materials umfasst.

7. Ballon (108; 400; 500; 550; 600; 700; 720; 740; 800; 810; 820; 830; 850; 860; 870; 880; 890; 900; 910; 920; 1101) gemäß Anspruch 4, wobei das zweite Segment (408; 508a, 508b; 558a, 558b; 608; 708; 808; 828; 838; 866; 898; 918; 932) eine sich in Umfangsrichtung erstreckende Schicht (422) des zweiten Materials umfasst, die zwischen mehreren sich in Umfangsrichtung erstreckenden Schichten (418, 420) des ersten Materials angeordnet ist.

8. Ballon (108; 400; 500; 550; 600; 700; 720; 740; 800; 810; 820; 830; 850; 860; 870; 880; 890; 900; 910; 920; 1101) gemäß Anspruch 3, wobei das erste Segment (406; 506a, 506b; 556a, 556b; 606; 706; 806; 826; 836; 852; 864; 884; 896; 916; 930) mehr sich in Umfangsrichtung erstreckende Schichten (418, 420) des ersten Materials als des zweiten Materials umfasst, und wobei das zweite Segment (408; 508a, 508b; 558a, 558b; 608; 708; 808; 828; 838; 866; 898; 918; 932) mehr sich in Umfangsrichtung erstreckende Schichten (422) des zweiten Materials umfasst als das erste Segment (406; 506a, 506b; 556a, 556b; 606; 706; 806; 826; 836; 852; 864; 884; 896; 916; 930).

9. Ballon (108; 400; 500; 550; 600; 700; 720; 740; 800; 810; 820; 830; 850; 860; 870; 880; 890; 900; 910; 920; 1101) gemäß einem der Ansprüche 1 bis 8, wobei der Ballon eine Mehrzahl von ersten Segmenten (406; 506a, 506b; 556a, 556b; 606; 706; 806; 826; 836; 852; 864; 884; 896; 916; 930) mit der ersten Nachgiebigkeit und eine Mehrzahl von zweiten Segmenten (408; 508a, 508b; 558a, 558b; 608; 708; 808; 828; 838; 866; 898; 918; 932) mit der zweiten Nachgiebigkeit umfasst, und wobei jedes erste Segment (406; 506a, 506b; 556a, 556b; 606; 706; 806; 826; 836; 852; 864; 884; 896; 916; 930) zwischen zwei benachbarten zweiten Segmenten (408; 508a, 508b; 558a, 558b; 608; 708; 808; 828; 838; 866; 898; 918; 932) in einer Umfangsrichtung des Ballons angeordnet ist.

10. Ballon (108; 400; 500; 550; 600; 700; 720; 740; 800; 810; 820; 830; 850; 860; 870; 880; 890; 900; 910; 920; 1101) gemäß Anspruch 1 oder Anspruch 2, wobei das erste Segment (406; 506a, 506b; 556a, 556b; 606; 706; 806; 826; 836; 852; 864; 884; 896; 916; 930) ein erstes Material mit einem ersten Härtegrad umfasst, das in ein zweites Material mit einem zweiten Härtegrad eingebettet ist, der größer ist als der erste Härtegrad, und wobei das zweite Segment (408; 508a, 508b; 558a, 558b; 608; 708; 808; 828; 838; 866; 898; 918; 932) das zweite Material umfasst.

11. Ballon (108; 400; 500; 550; 600; 700; 720; 740; 800; 810; 820; 830; 850; 860; 870; 880; 890; 900; 910; 920; 1101) gemäß Anspruch 1 oder Anspruch 2, wobei der Ballon einen ersten sich in Umfangsrichtung erstreckenden Abschnitt, umfassend ein erstes Material mit einem ersten Härtegrad, und einen zweiten sich in Umfangsrichtung erstreckenden Abschnitt, umfassend ein zweites Material mit einem zweiten Härtegrad, umfasst, wobei der zweite Härtegrad größer ist als der erste Härtegrad, und wobei das erste Segment (406; 506a, 506b; 556a, 556b; 606; 706; 806; 826; 836; 852; 864; 884; 896; 916; 930) durch eine Unterbrechung in dem zweiten sich in Umfangsrichtung erstreckenden Abschnitt gebildet ist, sodass eine Diskontinuität (710; 802; 881; 885; 886; 888; 892) in einer Umfangsrichtung gebildet ist.

12. Ballon (108; 400; 500; 550; 600; 700; 720; 740; 800; 810; 820; 830; 850; 860; 870; 880; 890; 900; 910; 920; 1101) gemäß einem der Ansprüche 1 bis 11, wobei der medizinische Katheter eine Zuführvorrichtung für eine radial expandierbare medizinische Vorrichtung ist.

13. Ballon (108; 400; 500; 550; 600; 700; 720; 740; 800; 810; 820; 830; 850; 860; 870; 880; 890; 900; 910; 920; 1101) gemäß einem der Ansprüche 1 bis 12, wobei eine Bogenlänge des zweiten Segments (408; 508a, 508b; 558a, 558b; 608; 708; 808; 828; 838; 866; 898; 918; 932) länger ist als eine Bogenlänge des ersten Segments (406; 506a, 506b; 556a, 556b; 606; 706; 806; 826; 836; 852; 864; 884; 896; 916; 930).

14. Ballon (108; 400; 500; 550; 600; 700; 720; 740; 800; 810; 820; 830; 850; 860; 870; 880; 890; 900; 910; 920; 1101) gemäß einem der Ansprüche 1 bis 13, wobei der medizinische Katheter (100) eine Zuführvorrichtung für eine radial expandierbare medizinische Vorrichtung ist, und wobei der Ballon (108; 400; 500; 550; 600; 700; 720; 740; 800; 810; 820; 830; 850; 860; 870; 880; 890; 900; 910; 920; 1101) dazu ausgelegt ist, an einem Schaft (104, 106) der Zuführvorrichtung befestigt zu werden.

15. Ballonkatheter (100), umfassend:
einen Schaft (104, 106), der sich von einem Griff (102) des Ballonkatheters erstreckt; und
den aufblasbaren Ballon (108; 400; 500; 550; 600; 700; 720; 740; 800; 810; 820; 830; 850; 860; 870; 880; 890; 900; 910; 920; 1101) gemäß mindestens einem der vorhergehenden Ansprüche, der an dem Schaft (104, 106) befestigt ist.

## Revendications

1. Ballonnet gonflable (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) pour un cathéter médical (100), comprenant :
un premier segment (406 ; 506a, 506b ; 556a, 556b ; 606 ; 706 ; 806 ; 826 ; 836 ; 852 ; 864 ; 884 ; 896 ; 916 ; 930) qui présente une première souplesse ; et
un second segment (408 ; 508a, 508b ; 558a, 558b ; 608 ; 708 ; 808 ; 828 ; 838 ; 866 ; 898 ; 918 ; 932) qui présente une seconde souplesse, la première souplesse étant supérieure à la seconde souplesse, le premier segment (406 ; 506a, 506b ; 556a, 556b ; 606 ; 706 ; 806 ; 826 ; 836 ; 852 ; 864 ; 884 ; 896 ; 916 ; 930) et le second segment (408 ; 508a, 508b ; 558a, 558b ; 608 ; 708 ; 808 ; 828 ; 838 ; 866 ; 898 ; 918 ; 932) s'étendant axialement le long d'une longueur du ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101), étant agencés de manière adjacente l'un à l'autre dans une direction circonférentielle du ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) et le premier segment (406 ; 506a, 506b ; 556a, 556b ; 606 ; 706 ; 806 ; 826 ; 836 ; 852 ; 864 ; 884 ; 896 ; 916 ; 930) étant conçu pour se rompre avant le second segment (408 ; 508a, 508b ; 558a, 558b ; 608 ; 708 ; 808 ; 828 ; 838 ; 866 ; 898 ; 918 ; 932) dans une direction axiale (124) sous la pression (612) d'un fluide de gonflage introduit dans le ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101).

2. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon la revendication 1, le premier segment (406 ; 506a, 506b ; 556a, 556b ; 606 ; 706 ; 806 ; 826 ; 836 ; 852 ; 864 ; 884 ; 896 ; 916 ; 930) et le second segment (408 ; 508a, 508b ; 558a, 558b ; 608 ; 708 ; 808 ; 828 ; 838 ; 866 ; 898 ; 918 ; 932) étant des segments circonférentiels du ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) qui s'étendent chacun dans une direction circonférentielle autour de différentes parties d'une circonférence du ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101).

3. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon soit la revendication 1, soit la revendication 2, le premier segment (406 ; 506a, 506b ; 556a, 556b ; 606 ; 706 ; 806 ; 826 ; 836 ; 852 ; 864 ; 884 ; 896 ; 916 ; 930) comprenant un premier matériau présentant la première souplesse et un premier duromètre et le second segment (408 ; 508a, 508b ; 558a, 558b ; 608 ; 708 ; 808 ; 828 ; 838 ; 866 ; 898 ; 918 ; 932) comprenant le premier matériau et un second matériau présentant un second duromètre qui est supérieur au premier duromètre.

4. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon la revendication 3, le premier segment (406 ; 506a, 506b ; 556a, 556b ; 606 ; 706 ; 806 ; 826 ; 836 ; 852 ; 864 ; 884 ; 896 ; 916 ; 930) comprenant une ou plusieurs couches (418, 420), s'étendant de manière circonférentielle, du premier matériau qui s'étendent sur une épaisseur du ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) et le second segment (408 ; 508a, 508b ; 558a, 558b ; 608 ; 708 ; 808 ; 828 ; 838 ; 866 ; 898 ; 918 ; 932) comprenant une ou plusieurs couches (418, 420), s'étendant de manière circonférentielle, du premier matériau et une ou plusieurs couches (422), s'étendant de manière circonférentielle, du second matériau.

5. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon la revendication 3, le premier segment (406 ; 506a, 506b ; 556a, 556b ; 606 ; 706 ; 806 ; 826 ; 836 ; 852 ; 864 ; 884 ; 896 ; 916 ; 930) comprenant une pluralité de couches (418, 420), s'étendant de manière circonférentielle, uniquement du premier matériau et le second segment (408 ; 508a, 508b ; 558a, 558b ; 608 ; 708 ; 808 ; 828 ; 838 ; 866 ; 898 ; 918 ; 932) comprenant une ou plusieurs couches (418, 420), s'étendant de manière circonférentielle, du premier matériau et une ou plusieurs couches (422), s'étendant de manière circonférentielle, du second matériau.

6. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon la revendication 3, le premier segment (406 ; 506a, 506b ; 556a, 556b ; 606 ; 706 ; 806 ; 826 ; 836 ; 852 ; 864 ; 884 ; 896 ; 916 ; 930) comprenant une pluralité de couches (418, 420), s'étendant de manière circonférentielle, du premier matériau et une seule couche (422), s'étendant de manière circonférentielle, du second matériau et le second segment (408 ; 508a, 508b ; 558a, 558b ; 608 ; 708 ; 808 ; 828 ; 838 ; 866 ; 898 ; 918 ; 932) comprenant une pluralité de couches (418, 420), s'étendant de manière circonférentielle, du premier matériau et au moins deux couches (422), s'étendant de manière circonférentielle, du second matériau.

7. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon la revendication 4, le second segment (408 ; 508a, 508b ; 558a, 558b ; 608 ; 708 ; 808 ; 828 ; 838 ; 866 ; 898 ; 918 ; 932) comprenant une couche (422), s'étendant de manière circonférentielle, du second matériau disposée entre de multiples couches (418, 420), s'étendant de manière circonférentielle, du premier matériau.

8. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon la revendication 3, le premier segment (406 ; 506a, 506b ; 556a, 556b ; 606 ; 706 ; 806 ; 826 ; 836 ; 852 ; 864 ; 884 ; 896 ; 916 ; 930) comprenant plus de couches (418, 420), s'étendant de manière circonférentielle, du premier matériau que du second matériau et le second segment (408 ; 508a, 508b ; 558a, 558b ; 608 ; 708 ; 808 ; 828 ; 838 ; 866 ; 898 ; 918 ; 932) comprenant plus de couches (422), s'étendant de manière circonférentielle, du second matériau que le premier segment (406 ; 506a, 506b ; 556a, 556b ; 606 ; 706 ; 806 ; 826 ; 836 ; 852 ; 864 ; 884 ; 896 ; 916 ; 930).

9. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon l'une quelconque des revendications 1 à 8, le ballonnet comprenant une pluralité de premiers segments (406 ; 506a, 506b ; 556a, 556b ; 606 ; 706 ; 806 ; 826 ; 836 ; 852 ; 864 ; 884 ; 896 ; 916 ; 930) présentant la première souplesse et une pluralité de seconds segments (408 ; 508a, 508b ; 558a, 558b ; 608 ; 708 ; 808 ; 828 ; 838 ; 866 ; 898 ; 918 ; 932) présentant la seconde souplesse et chaque premier segment (406 ; 506a, 506b ; 556a, 556b ; 606 ; 706 ; 806 ; 826 ; 836 ; 852 ; 864 ; 884 ; 896 ; 916 ; 930) étant disposé entre deux seconds segments adjacents (408 ; 508a, 508b ; 558a, 558b ; 608 ; 708 ; 808 ; 828 ; 838 ; 866 ; 898 ; 918 ; 932) dans une direction circonférentielle du ballonnet.

10. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon soit la revendication 1, soit la revendication 2, le premier segment (406 ; 506a, 506b ; 556a, 556b ; 606 ; 706 ; 806 ; 826 ; 836 ; 852 ; 864 ; 884 ; 896 ; 916 ; 930) comprenant un premier matériau présentant un premier duromètre incorporé dans un second matériau présentant un second duromètre qui est supérieur au premier duromètre et le second segment (408 ; 508a, 508b ; 558a, 558b ; 608 ; 708 ; 808 ; 828 ; 838 ; 866 ; 898 ; 918 ; 932) comprenant le second matériau.

11. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon soit la revendication 1, soit la revendication 2, le ballonnet comprenant une première partie s'étendant de manière circonférentielle comprenant un premier matériau présentant un premier duromètre et une seconde partie s'étendant de manière circonférentielle comprenant un second matériau présentant un second duromètre, le second duromètre étant supérieur au premier duromètre, et le premier segment (406 ; 506a, 506b ; 556a, 556b ; 606 ; 706 ; 806 ; 826 ; 836 ; 852 ; 864 ; 884 ; 896 ; 916 ; 930) étant formé par une rupture dans la seconde partie s'étendant de manière circonférentielle de telle sorte qu'une discontinuité (710 ; 802 ; 881 ; 885 ; 886 ; 888 ; 892) est formée dans une direction circonférentielle.

12. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon l'une quelconque des revendications 1 à 11, le cathéter médical étant un appareil de pose pour un dispositif médical radialement déployable.

13. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon l'une quelconque des revendications 1 à 12, une longueur d'arc du second segment (408 ; 508a, 508b ; 558a, 558b ; 608 ; 708 ; 808 ; 828 ; 838 ; 866 ; 898 ; 918 ; 932) étant plus longue qu'une longueur d'arc du premier segment (406 ; 506a, 506b ; 556a, 556b ; 606 ; 706 ; 806 ; 826 ; 836 ; 852 ; 864 ; 884 ; 896 ; 916 ; 930).

14. Ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon l'une quelconque des revendications 1 à 13, le cathéter médical (100) étant un appareil de pose pour un dispositif médical radialement déployable et le ballonnet (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) étant conçu pour être fixé à une tige (104, 106) de l'appareil de pose.

15. Cathéter (100) à ballonnet comprenant :
une tige (104, 106) s'étendant à partir d'une poignée (102) du cathéter à ballonnet ; et
le ballonnet gonflable (108 ; 400 ; 500 ; 550 ; 600 ; 700 ; 720 ; 740 ; 800 ; 810 ; 820 ; 830 ; 850 ; 860 ; 870 ; 880 ; 890 ; 900 ; 910 ; 920 ; 1101) selon au moins l'une des revendications précédentes monté sur la tige (104, 106).
